# EUROPEAN PATENT APPLICATION

(11) **EP 4 582 085 A1**
(43) Date of publication of application: **09.07.2025**
(21) Application number: 23859436.0
(22) Date of filing: 31.08.2023
(51) Int. Cl.: A61K 31/496, A61K 31/444, C07D 401/12, C07D 213/75, C07D 213/63, A61P 35/00

(54) **USE OF HETEROARYLOXYNAPHTHALENE COMPOUND**

(30) Priority: 31.08.2022 CN 202211062094
(71) Applicant: Shanghai Runshi Medical Technology Co., Ltd, Shanghai 200040 (CN); Shanghai Institute of Materia Medica, Chinese Academy of Sciences, Shanghai 201203 (CN)
(72) Inventor: LIU, Xiaowen, Shanghai 200040 (CN); XU, Wen, Shanghai 200040 (CN); WANG, Juan, Shanghai 200040 (CN); ZHANG, Ao, Shanghai 201203 (CN); GENG, Meiyu, Shanghai 201203 (CN); SONG, Zilan, Shanghai 201203 (CN); AI, Jing, Shanghai 201203 (CN); YANG, Hanyu, Shanghai 200040 (CN); ZHANG, Yang, Shanghai 200040 (CN); HAO, Hongru, Shanghai 200040 (CN)
(74) Representative: Gille Hrabal Partnerschaftsgesellschaft mbB Patentanwälte
(86) International application number: PCT/CN2023/116023
(87) International publication number: WO 2024/046407

(57) **Abstract**

Provided is use of a heteroaryloxynaphthalene compound, in particular, use of compound A or a pharmaceutically acceptable salt thereof in preparing a medicament for treating a tumor-related disease. Compound A or the pharmaceutically acceptable salt thereof exhibits good efficacy against tumors and good tolerance, and thus has clinical potential for treating tumors.

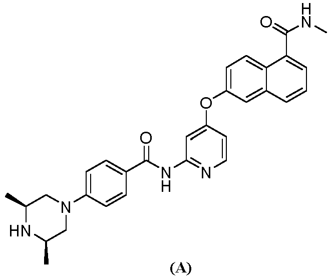

## Description

The present application claims priority to the prior application with the patent application No. 202211062094.6 and entitled "USE OF HETEROARYLOXYNAPHTHALENE COMPOUND" filed with the China National Intellectual Property Administration on August 31, 2022, the content of which is incorporated herein by reference in its entirety.

### TECHNICAL FIELD

The present disclosure relates to the field of pharmaceuticals, and in particular to use of a multi-target protein kinase inhibitor in the manufacture of a medicament for treating and/or preventing a tumor, particularly squamous cell carcinoma, esophageal cancer, and/or carcinoma at the esophagogastric junction.

### BACKGROUND

Squamous cell carcinoma (SCC), also known as epidermoid carcinoma, refers to various types of cancer arising from squamous cells. These cells are formed on the surface of the skin, the inner wall of hollow organs in the body, and the inner wall of the respiratory and digestive tracts. For example, depending on the location in the body, squamous cell carcinoma can occur in the skin, head and neck, digestive tract (e.g., esophagus), respiratory tract (e.g., lungs), etc.

Esophageal cancer (or esophagus cancer) is one of the most common malignant tumors worldwide, ranking 8th in incidence and 6th in mortality among cancer types. Moreover, compared with the general population, individuals with esophageal cancer have an increased risk of developing multiple primary malignant tumors, with an incidence rate as high as 9.5% to 21.9%. From a histological perspective, esophageal cancer is mainly divided into two types: squamous cell carcinoma (esophageal squamous cell carcinoma) and adenocarcinoma (esophageal adenocarcinoma).

The clinical symptoms of early-stage esophageal cancer are not obvious, making it difficult to be detected. Most esophageal cancer patients are already at the locally advanced stage or have distant metastases at the time of diagnosis. Although methods such as surgical therapy, endoscopic therapy, radiotherapy, chemotherapy, immunotherapy, and the like are already in clinical use, patients still face the problem of poor prognosis, with the overall 5-year survival rate being only 10%-20%.

According to the Chinese Society of Clinical Oncology (CSCO) Guidelines for the Diagnosis and Treatment of Esophageal Cancer (2022 edition), the first-line treatment for advanced esophageal cancer primarily involves chemotherapy in combination with immunotherapy and chemotherapy. For patients stratified as squamous cell carcinoma and HER-2 negative adenocarcinoma (PS = 0-2), in the recommended second-line and later-line treatments, single agent chemotherapy and single agent immunotherapy are primarily given as a Grade I recommendation for cases stratified as PS = 0-2. Currently, no small molecule targeted drugs for the treatment of esophageal cancer have been approved for marketing. Moreover, among the chemotherapeutic drugs recommended by the guidelines for the treatment of advanced esophageal cancer, platinum-containing and fluorouracil-containing drugs are commonly used, but they are often accompanied by cytotoxic side effects. Meanwhile, many studies have shown that the incidence of esophageal cancer-related multiple primary malignant tumors is significantly associated with the effects of genetic factors, iatrogenic factors (e.g., radiotherapy, chemotherapy, or the like), etc. The common radiotherapy and chemotherapy in the treatment of esophageal cancer, while improving patient survival rates, may also increase the risk of developing related multiple primary malignant tumors. Therefore, for esophageal cancer patients who have failed existing treatments, there is currently no standard treatment regimen for later-line treatment. The efficacy of treatment methods with the same or similar mechanisms of action is difficult to be guaranteed. Patients who have experienced disease progression after prior front-line treatments are in urgent need of effective therapeutic drugs to address the unmet clinical needs.

CN108699030A discloses compound A (corresponding to compound S10), which is a compound with inhibitory activity against fibroblast growth factor receptors (FGFRs) and vascular endothelial growth factor receptors (KDR or VEGFR2). Currently, there are no relevant research reports on its potential use for the treatment of squamous cell carcinoma, digestive tract squamous cell carcinoma, esophageal cancer, carcinoma at the esophagogastric junction, etc.

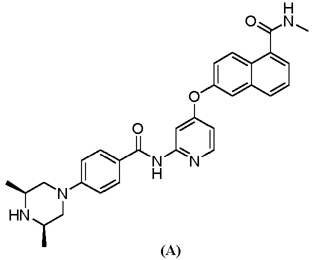

### SUMMARY

The technical problem to be solved by the present application is to provide use of compound A or a pharmaceutically acceptable salt thereof in the manufacture of a medicament for treating a tumor, particularly squamous cell carcinoma, digestive tract squamous cell carcinoma, esophageal cancer, or carcinoma at the esophagogastric junction.

Specifically, in a first aspect, the present application provides use of compound A or a pharmaceutically acceptable salt thereof in the manufacture of a medicament for treating a tumor, wherein the tumor is esophageal cancer or carcinoma at the esophagogastric junction,

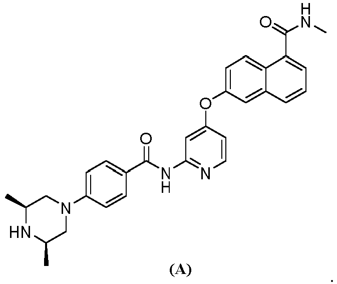

In some embodiments of the first aspect of the present application, the carcinoma at the esophagogastric junction is squamous cell carcinoma at the esophagogastric junction or adenocarcinoma at the esophagogastric junction, preferably squamous cell carcinoma at the esophagogastric junction.

In some embodiments of the first aspect of the present application, the esophageal cancer is esophageal squamous cell carcinoma or esophageal adenocarcinoma, preferably esophageal squamous cell carcinoma.

In some embodiments of the first aspect of the present application, the tumor is a tumor with a genetic abnormality and/or abnormal protein expression of FGFR and/or VEGFR.

In some embodiments of the first aspect of the present application, the tumor is a tumor with a genetic abnormality and/or abnormal protein expression of one, two, or three of FGFR, VEGFR, and colony-stimulating factor 1 receptor (CSF1R). Preferably, the tumor is a tumor with a gene mutation in one, two, or three of FGFR, VEGFR, and CSF1R. In some embodiments of the first aspect of the present application, the tumor is a locally advanced tumor or a metastatic tumor, preferably an unresectable locally advanced tumor or an unresectable metastatic tumor.

In some embodiments of the first aspect of the present application, the tumor is a tumor that has failed a prior treatment.

In some embodiments of the first aspect of the present application, the tumor is a tumor that has failed a systemic drug treatment.

In some embodiments of the first aspect of the present application, the tumor is a tumor that has failed a first-line treatment.

In some embodiments of the first aspect of the present application, the tumor is a tumor that has failed a second-line treatment.

In some embodiments of the first aspect of the present application, the tumor is a tumor that has failed a later-line treatment.

In some embodiments of the first aspect of the present application, the tumor is a tumor that has failed treatment with one, two, three, or more of an immunotherapeutic drug, a chemotherapeutic drug, radiotherapy, biological therapy, traditional Chinese medicine therapy, and a small molecule targeted drug.

In some embodiments of the first aspect of the present application, the tumor is a tumor that has failed treatment with radiotherapy, an immunotherapeutic drug, or a chemotherapeutic drug. In some embodiments of the first aspect of the present application, the tumor is a tumor that has failed treatment with radiotherapy and an immunotherapeutic drug. In some embodiments of the first aspect of the present application, the tumor is a tumor that has failed treatment with radiotherapy and a chemotherapeutic drug. In some embodiments of the first aspect of the present application, the tumor is a tumor that has failed treatment with a chemotherapeutic drug and an immunotherapeutic drug. In some embodiments of the first aspect of the present application, the tumor is a tumor that has failed treatment with a chemotherapeutic drug, an immunotherapeutic drug, and radiotherapy.

In some embodiments of the first aspect of the present application, the esophageal squamous cell carcinoma is esophageal squamous cell carcinoma with a genetic abnormality or abnormal protein expression of FGFR and/or VEGFR.

In some embodiments of the first aspect of the present application, the esophageal squamous cell carcinoma is esophageal squamous cell carcinoma with a genetic abnormality and/or abnormal protein expression of one, two, or three of FGFR, VEGFR, and colony-stimulating factor 1 receptor (CSF1R). Preferably, the esophageal squamous cell carcinoma is esophageal squamous cell carcinoma with a gene mutation in one, two, or three of FGFR, VEGFR, and CSF1R.

In some embodiments of the first aspect of the present application, the esophageal squamous cell carcinoma is locally advanced esophageal squamous cell carcinoma or metastatic (e.g. stage IV) esophageal squamous cell carcinoma, preferably an unresectable locally advanced esophageal squamous cell carcinoma or an unresectable metastatic esophageal squamous cell carcinoma.

In some embodiments of the first aspect of the present application, the esophageal squamous cell carcinoma is an esophageal squamous cell carcinoma that has failed a prior treatment.

In some embodiments of the first aspect of the present application, the esophageal squamous cell carcinoma is an esophageal squamous cell carcinoma that has failed a systemic drug treatment.

In some embodiments of the first aspect of the present application, the esophageal squamous cell carcinoma is an esophageal squamous cell carcinoma that has failed a first-line treatment.

In some embodiments of the first aspect of the present application, the esophageal squamous cell carcinoma is an esophageal squamous cell carcinoma that has failed a second-line treatment.

In some embodiments of the first aspect of the present application, the esophageal squamous cell carcinoma is an esophageal squamous cell carcinoma that has failed a later-line treatment.

In some embodiments of the first aspect of the present application, the esophageal squamous cell carcinoma is an esophageal squamous cell carcinoma that has failed treatment with one, two, three, or more of an immunotherapeutic drug, a chemotherapeutic drug, radiotherapy, biological therapy, traditional Chinese medicine therapy, and a small molecule targeted drug. In some embodiments of the first aspect of the present application, the esophageal squamous cell carcinoma is an esophageal squamous cell carcinoma that has failed treatment with radiotherapy, an immunotherapeutic drug, or a chemotherapeutic drug. In some embodiments of the first aspect of the present application, the esophageal squamous cell carcinoma is an esophageal squamous cell carcinoma that has failed treatment with radiotherapy and an immunotherapeutic drug. In some embodiments of the first aspect of the present application, the esophageal squamous cell carcinoma is an esophageal squamous cell carcinoma that has failed treatment with radiotherapy and a chemotherapeutic drug. In some embodiments of the first aspect of the present application, the esophageal squamous cell carcinoma is an esophageal squamous cell carcinoma that has failed treatment with a chemotherapeutic drug and an immunotherapeutic drug. In some embodiments of the first aspect of the present application, the esophageal squamous cell carcinoma is an esophageal squamous cell carcinoma that has failed treatment with a chemotherapeutic drug, immunotherapy, and radiotherapy.

In some embodiments of the first aspect of the present application, the aforementioned chemotherapeutic drug is selected from a cytotoxic drug, an antimetabolite drug, an antibiotic drug, an alkaloid drug, a DNA topoisomerase inhibitor chemotherapeutic drug, a microtubule-targeting chemotherapeutic drug, a platinum-based chemotherapeutic drug, and a hormone drug; preferably, the aforementioned chemotherapeutic drug is selected from an antimetabolite drug, an alkaloid drug, a DNA topoisomerase inhibitor chemotherapeutic drug, a microtubule-targeting chemotherapeutic drug, and a platinum-based chemotherapeutic drug.

In some embodiments of the first aspect of the present application, the aforementioned chemotherapeutic drug is selected from a taxane-based drug, a platinum-based drug, a fluorouracil-based drug, and a DNA topoisomerase inhibitor chemotherapeutic drug. In some embodiments of the first aspect of the present application, the taxane-based drug is selected from paclitaxel, docetaxel, and cabazitaxel, preferably paclitaxel. In some embodiments of the first aspect of the present application, the DNA topoisomerase inhibitor chemotherapeutic drug is selected from hydroxycamptothecin, irinotecan, topotecan, etoposide, and teniposide. In some embodiments of the first aspect of the present application, the platinum-based drug is selected from cisplatin, carboplatin, sulfatodiamino cyclohexane platinum, lobaplatin, oxaliplatin, and nedaplatin, preferably cisplatin, oxaliplatin, or nedaplatin. In some embodiments of the first aspect of the present application, the aforementioned chemotherapeutic drug is selected from paclitaxel, cisplatin, and nedaplatin.

In some embodiments of the first aspect of the present application, the aforementioned immunotherapeutic drug is selected from an anti-programmed death receptor 1 antibody (anti-PD-1 antibody), an anti-programmed death receptor-ligand 1 antibody (anti-PD-L1 antibody), and an antibody-drug conjugate. Preferably, the antibody-drug conjugate is an anti-HER2 antibody-drug conjugate. In some embodiments of the first aspect of the present application, the anti-PD-1 antibody is selected from pembrolizumab, camrelizumab, tislelizumab, nivolumab, cemiplimab, sintilimab, and toripalimab; preferably, the anti-PD-1 antibody is selected from toripalimab, sintilimab, and tislelizumab. In some embodiments of the first aspect of the present application, the anti-PD-L1 antibody is selected from atezolizumab, avelumab, and durvalumab. In some embodiments of the first aspect of the present application, the anti-HER2 antibody-drug conjugate is selected from trastuzumab and disitamab.

In some embodiments of the first aspect of the present application, the tumor is a tumor that has failed treatment with a chemotherapeutic drug and an immunotherapeutic drug; preferably, the tumor that has failed treatment is a tumor that has failed treatment with a taxane-based chemotherapeutic drug + a platinum-based chemotherapeutic drug + an anti-PD-1 antibody; preferably, the tumor that has failed treatment is a tumor that has failed treatment with one, two, or three of toripalimab + paclitaxel + cisplatin, sintilimab + paclitaxel + cisplatin, paclitaxel + lobaplatin + camrelizumab, paclitaxel + nedaplatin, and sintilimab. Preferably, the tumor that has failed treatment is a tumor that has failed treatment with one, two, or three of toripalimab + paclitaxel + cisplatin, sintilimab + paclitaxel + cisplatin, and paclitaxel + lobaplatin + camrelizumab. Preferably, the tumor is esophageal squamous cell carcinoma; preferably, the tumor is squamous cell carcinoma at the esophagogastric junction.

In some embodiments of the first aspect of the present application, the tumor is a tumor that has failed treatment with a chemotherapeutic drug; preferably, the tumor is a tumor that has failed treatment with a taxane-based chemotherapeutic drug + a platinum-based chemotherapeutic drug; preferably, the tumor that has failed treatment is a tumor that has failed treatment with paclitaxel + cisplatin or paclitaxel + nedaplatin. Preferably, the tumor is esophageal squamous cell carcinoma.

In some embodiments of the first aspect of the present application, the tumor is a tumor that has failed treatment with a chemotherapeutic drug, immunotherapy, and radiotherapy; preferably, the tumor is a tumor that has failed treatment with a taxane-based chemotherapeutic drug + a platinum-based chemotherapeutic drug + radiotherapy + an anti-PD-1 antibody; preferably, the tumor that has failed treatment is a tumor that has failed treatment with tislelizumab + radiotherapy + paclitaxel + cisplatin. Preferably, the tumor is esophageal squamous cell carcinoma. In some embodiments of the first aspect of the present application, the pharmaceutically acceptable salt is selected from hydrochloride, hydrobromide, nitrate, sulfate, phosphate, formate, acetate, propionate, benzoate, maleate, fumarate, succinate, tartrate, citrate, methanesulfonate, ethanesulfonate, benzenesulfonate, and p-toluenesulfonate, preferably hydrochloride, hydrobromide, maleate, phosphate, tartrate, fumarate, and succinate, further preferably hydrochloride, maleate, phosphate, tartrate, and fumarate, further preferably hydrochloride, and still further preferably dihydrochloride.

In some embodiments of the first aspect of the present application, the pharmaceutically acceptable salt of compound A is compound B:

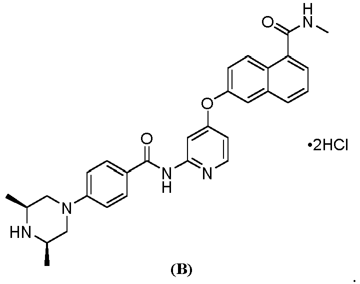

In some embodiments of the first aspect of the present application, the compound B is in a solid form, preferably in a crystalline form.

In some embodiments of the first aspect of the present application, the crystalline form of compound B is a crystalline form I having characteristic peaks in an X-ray powder diffraction pattern using Cu-Kα radiation at the following 2θ angles: 5.0±0.2°, 13.5±0.2°, 19.3±0.2°, 20.5±0.2°, 21.4±0.2°, and 25.2±0.2°.

In some embodiments of the first aspect of the present application, the crystalline form I has characteristic peaks in an X-ray powder diffraction pattern using Cu-Kα radiation at the following 20 angles: 5.0±0.2°, 13.5±0.2°, 19.3±0.2°, 20.5±0.2°, 21.4±0.2°, 22.5±0.2°, 23.4±0.2°, and 25.2±0.2°.

In some embodiments of the first aspect of the present application, the crystalline form I has characteristic peaks in an X-ray powder diffraction pattern using Cu-Kα radiation at the following 20 angles: 5.0±0.2°, 13.5±0.2°, 19.3±0.2°, 20.5±0.2°, 21.4±0.2°, 22.5±0.2°, 23.4±0.2°, 23.9±0.2°, 24.3±0.2°, and 25.2±0.2°.

In some embodiments of the first aspect of the present application, the crystalline form I has characteristic peaks in an X-ray powder diffraction pattern using Cu-Kα radiation at the following 20 angles: 5.0±0.2°, 10.4±0.2°, 11.2±0.2°, 13.5±0.2°, 19.3±0.2°, 20.5±0.2°, 21.4±0.2°, 22.0±0.2°, 22.5±0.2°, 23.4±0.2°, 23.9±0.2°, 24.3±0.2°, 25.2±0.2°, and 26.6±0.2°.

In some embodiments of the first aspect of the present application, the crystalline form I has characteristic peaks in an X-ray powder diffraction pattern using Cu-Kα radiation at the following 20 angles: 5.0±0.2°, 10.0±0.2°, 10.4±0.2°, 11.2±0.2°, 13.5±0.2°, 14.1±0.2°, 15.7±0.2°, 17.4±0.2°, 18.0±0.2°, 19.3±0.2°, 20.1±0.2°, 20.5±0.2°, 21.4±0.2°, 22.0±0.2°, 22.5±0.2°, 23.4±0.2°, 23.9±0.2°, 24.3±0.2°, 25.2±0.2°, 26.6±0.2°, 28.6±0.2°, and 30.1±0.2°. In some embodiments of the first aspect of the present application, the compound A or the pharmaceutically acceptable salt thereof is administered via a pharmaceutically acceptable route, including but not limited to a route selected from: oral administration, parenteral administration, and topical administration. In a particular embodiment, administration is carried out orally.

In some embodiments of the first aspect of the present application, the medicament is formulated into a clinically acceptable formulation, including but not limited to an oral formulation, a parenteral formulation, a topical formulation, or an external formulation, preferably an oral formulation, and further preferably a tablet and a capsule. In some embodiments of the first aspect of the present application, each unit of formulation of the medicament comprises the compound A or the pharmaceutically acceptable salt thereof in an amount of 0.1-200 mg, preferably 1-150 mg, or 1-100 mg, or 1-80 mg, or 1-60 mg, or 1-50 mg, or 1-40 mg, or 1-30 mg, or 1-20 mg, or 1-10 mg, or 1-6 mg, such as 1 mg, 2 mg, 3 mg, 4 mg, 5 mg, 6 mg, 8 mg, 10 mg, 15 mg, 20 mg, or 25 mg. In some embodiments of the first aspect of the present application, each unit of formulation of the medicament comprises the compound A or the pharmaceutically acceptable salt thereof in an amount of 1 mg, 2 mg, 3 mg, 4 mg, 5 mg, 6 mg, 7 mg, 8 mg, 9 mg, 10 mg, 15 mg, 20 mg, or 25 mg. In some embodiments of the first aspect of the present application, each unit of formulation of the medicament comprises the compound A or the pharmaceutically acceptable salt thereof in an amount of 1 mg, 2 mg, 3 mg, 4 mg, 5 mg, 6 mg, 7 mg, 8 mg, or 9 mg. Preferably, the pharmaceutically acceptable salt of the compound A is the compound B. Further preferably, the amount is calculated based on the mass of the compound B contained.

In some embodiments of the first aspect of the present application, the compound A or the pharmaceutically acceptable salt thereof is administered in a single dose or in divided doses, preferably in a single dose; preferably, the pharmaceutically acceptable salt of the compound A is the compound B; further preferably, it is administered in the form of the compound B.

In some embodiments of the first aspect of the present application, the administration dose of the compound A or the pharmaceutically acceptable salt thereof is determined based on the severity of the disease, the response to the disease, any treatment-related toxicity, and the age and health condition of the patient. In some embodiments of the first aspect of the present application, the compound A or the pharmaceutically acceptable salt thereof is administered at a daily dose of 1-50 mg. Preferably, the compound A or the pharmaceutically acceptable salt thereof is administered at a daily dose of 1-30 mg. Preferably, the compound A or the pharmaceutically acceptable salt thereof is administered at a daily dose of 1-25 mg. Preferably, the compound A or the pharmaceutically acceptable salt thereof is administered at a daily dose of 1-20 mg. Preferably, the compound A or the pharmaceutically acceptable salt thereof is administered at a daily dose of 2-18 mg. Preferably, the compound A or the pharmaceutically acceptable salt thereof is administered at a daily dose of 4-16 mg. Preferably, the compound A or the pharmaceutically acceptable salt thereof is administered at a daily dose of 5-15 mg. Preferably, the compound A or the pharmaceutically acceptable salt thereof is administered at a daily dose of 4-10 mg. Preferably, the compound A or the pharmaceutically acceptable salt thereof is administered at a daily dose of 4-9 mg. Preferably, the compound A or the pharmaceutically acceptable salt thereof is administered at a daily dose of 2-9 mg. Preferably, the compound A or the pharmaceutically acceptable salt thereof is administered at a daily dose of 2-6 mg. Preferably, the compound A or the pharmaceutically acceptable salt thereof is administered at a daily dose of 5-9 mg. Preferably, the compound A or the pharmaceutically acceptable salt thereof is administered at a daily dose of 6-9 mg. Preferably, the compound A or the pharmaceutically acceptable salt thereof is administered at a daily dose of 6-8 mg. Preferably, the compound A or the pharmaceutically acceptable salt thereof is administered at a daily dose of 6-12 mg. Preferably, the compound A or the pharmaceutically acceptable salt thereof is administered at a daily dose of 6-10 mg. Preferably, the compound A or the pharmaceutically acceptable salt thereof is administered at a daily dose of 2 mg, 3 mg, 4 mg, 5 mg, 6 mg, 7 mg, 8 mg, 9 mg, or 10 mg. Preferably, the compound A or the pharmaceutically acceptable salt thereof is administered at a daily dose of 2 mg, 4 mg, 5 mg, 6 mg, 7 mg, 8 mg, 9 mg, or 10 mg. Preferably, the compound A or the pharmaceutically acceptable salt thereof is administered at a daily dose of 6 mg, 7 mg, 8 mg, or 9 mg. Preferably, the compound A or the pharmaceutically acceptable salt thereof is administered at a daily dose of 4 mg. Preferably, the compound A or the pharmaceutically acceptable salt thereof is administered at a daily dose of 5 mg. Preferably, the compound A or the pharmaceutically acceptable salt thereof is administered at a daily dose of 6 mg. Preferably, the compound A or the pharmaceutically acceptable salt thereof is administered at a daily dose of 7 mg. Preferably, the compound A or the pharmaceutically acceptable salt thereof is administered at a daily dose of 8 mg. Preferably, the compound A or the pharmaceutically acceptable salt thereof is administered at a daily dose of 9 mg. Preferably, the pharmaceutically acceptable salt of the compound A is the compound B. Further preferably, the compound B is given in the form of an oral solid formulation containing the compound B. Further preferably, the daily dose is calculated based on the mass of the compound B contained.

In some embodiments of the first aspect of the present application, the compound A or the pharmaceutically acceptable salt thereof is administered at a frequency of once, twice, three times, or more times daily. In some embodiments of the first aspect of the present application, the compound A or the pharmaceutically acceptable salt thereof is administered once daily. In some embodiments of the first aspect of the present application, the compound A or the pharmaceutically acceptable salt thereof is administered twice daily. In some embodiments of the first aspect of the present application, the compound A or the pharmaceutically acceptable salt thereof is administered three times daily. Preferably, the pharmaceutically acceptable salt of the compound A is the compound B. Further preferably, the compound B is given in the form of an oral solid formulation containing the compound B.

In some embodiments of the first aspect of the present application, the method of administration is comprehensively determined based on the activity and toxicity of the medicament, the tolerance of the patient, etc. In some embodiments of the first aspect of the present application, the compound A or the pharmaceutically acceptable salt thereof is given according to a continuous administration regimen or an intermittent administration regimen. Preferably, the intermittent administration regimen comprises an administration period and an interruption period; preferably, the compound A or the pharmaceutically acceptable salt thereof is administered once, twice, three times, or more times daily during the administration period. For example, the compound A or the pharmaceutically acceptable salt thereof is administered once daily during the administration period, and then the administration is interrupted for a period of time during the interruption period, followed by another administration period and then another interruption period; such a cycle is repeated multiple times. The ratio of the number of days in the administration period to the interruption period is 3:1-5, preferably 3:1-3, more preferably 3:1-2, and even more preferably 3:1 or 3:1.5 (i.e., 2:1). Preferably, the pharmaceutically acceptable salt of the compound A is the compound B. Further preferably, the compound B is given in the form of an oral solid formulation containing the compound B.

In some embodiments of the first aspect of the present application, the compound A or the pharmaceutically acceptable salt thereof is administered according to a method as follows: continuous administration, once daily. In some embodiments of the first aspect of the present application, the compound A or the pharmaceutically acceptable salt thereof is administered according to a method as follows: continuous administration, twice daily. In some embodiments of the first aspect of the present application, the compound A or the pharmaceutically acceptable salt thereof is administered according to a method as follows: continuous administration, 3 times daily. Preferably, the pharmaceutically acceptable salt of the compound A is the compound B. Further preferably, the compound B is given in the form of an oral solid formulation containing the compound B.

In some embodiments of the first aspect of the present application, the compound A or the pharmaceutically acceptable salt thereof is administered according to a method as follows: 3-week continuous administration, followed by 1-week interruption. In some embodiments of the first aspect of the present application, the compound A or the pharmaceutically acceptable salt thereof is administered once daily for 21 consecutive days, followed by 7-day interruption, and then administered once daily for another 21 consecutive days, followed by another 7-day interruption; such an intermittent administration regimen of 3-week continuous administration and then 1-week interruption can be repeated multiple times. In some embodiments of the first aspect of the present application, the compound A or the pharmaceutically acceptable salt thereof is administered twice daily for 21 consecutive days, followed by 7-day interruption, and then administered twice daily for another 21 consecutive days, followed by another 7-day interruption; such an intermittent administration regimen of 3-week continuous administration and then 1-week interruption can be repeated multiple times. In some embodiments of the first aspect of the present application, the compound A or the pharmaceutically acceptable salt thereof is administered 3 times daily for 21 consecutive days, followed by 7-day interruption, and then administered 3 times daily for another 21 consecutive days, followed by another 7-day interruption; such an intermittent administration regimen of 3-week continuous administration and then 1-week interruption can be repeated multiple times. Preferably, the pharmaceutically acceptable salt of the compound A is the compound B. Further preferably, the compound B is given in the form of an oral solid formulation containing the compound B.

In some embodiments of the first aspect of the present application, the compound A or the pharmaceutically acceptable salt thereof is administered according to a method as follows: 2-week continuous administration, followed by 1-week interruption. In some embodiments, the compound A or the pharmaceutically acceptable salt thereof is administered once daily for 14 consecutive days, followed by 7-day interruption, and then administered once daily for another 14 consecutive days, followed by another 7-day interruption; such an intermittent administration regimen of 2-week continuous administration and then 1-week interruption can be repeated multiple times. In some embodiments, the compound A or the pharmaceutically acceptable salt thereof is administered twice daily for 14 consecutive days, followed by 7-day interruption, and then administered twice daily for another 14 consecutive days, followed by another 7-day interruption; such an intermittent administration regimen of 2-week continuous administration and then 1-week interruption can be repeated multiple times. In some embodiments, the compound A or the pharmaceutically acceptable salt thereof is administered 3 times daily for 14 consecutive days, followed by 7-day interruption, and then administered 3 times daily for another 14 consecutive days, followed by another 7-day interruption; such an intermittent administration regimen of 2-week continuous administration and then 1-week interruption can be repeated multiple times. Preferably, the pharmaceutically acceptable salt of the compound A is the compound B. Further preferably, the compound B is given in the form of an oral solid formulation containing the compound B.

In some embodiments of the first aspect of the present application, the compound A or the pharmaceutically acceptable salt thereof is administered according to a method as follows: 3-week continuous administration, followed by 2-week interruption. In some embodiments of the first aspect of the present application, the compound A or the pharmaceutically acceptable salt thereof is administered m times daily for 21 consecutive days, followed by 14-day interruption, and then administered m times daily for another 21 consecutive days, followed by another 14-day interruption; such an intermittent administration regimen of 3-week continuous administration and then 2-week interruption can be repeated multiple times. Preferably, the pharmaceutically acceptable salt of the compound A is the compound B. Further preferably, the compound B is given in the form of an oral solid formulation containing the compound B.

In some embodiments of the first aspect of the present application, the compound A or the pharmaceutically acceptable salt thereof is administered according to a method as follows: 5-day continuous administration, followed by 2-day interruption. In some embodiments, the compound A or the pharmaceutically acceptable salt thereof is administered m times daily for 5 consecutive days, followed by 2-day interruption, and then administered m times daily for another 5 consecutive days, followed by another 2-day interruption; such an intermittent administration regimen of 5-day continuous administration and then 2-day interruption can be repeated multiple times. Preferably, the pharmaceutically acceptable salt of the compound A is the compound B. Further preferably, the compound B is given in the form of an oral solid formulation containing the compound B.

In some embodiments of the first aspect of the present application, the compound A or the pharmaceutically acceptable salt thereof is administered according to a method as follows: continuous oral administration at a dose of n mg per time, m times daily. Preferably, the pharmaceutically acceptable salt of the compound A is the compound B. Further preferably, the compound B is given in the form of an oral solid formulation containing the compound B. Further preferably, the dose is calculated based on the mass of the compound B.

In some embodiments of the first aspect of the present application, the compound A or the pharmaceutically acceptable salt thereof is administered according to a method as follows: 3-week continuous oral administration at a dose of n mg per time, m times daily, followed by 1-week interruption, with 4 weeks constituting one cycle. Preferably, the pharmaceutically acceptable salt of the compound A is the compound B. Further preferably, the compound B is given in the form of an oral solid formulation containing the compound B. Further preferably, the dose is calculated based on the mass of the compound B.

In some embodiments of the first aspect of the present application, the compound A or the pharmaceutically acceptable salt thereof is administered according to a method as follows: 2-week continuous oral administration at a dose of n mg per time, m times daily, followed by 1-week interruption, with 3 weeks constituting 1 cycle. Preferably, the pharmaceutically acceptable salt of the compound A is the compound B. Further preferably, the compound B is given in the form of an oral solid formulation containing the compound B. Further preferably, the dose is calculated based on the mass of the compound B.

In some embodiments of the first aspect of the present application, the compound A or the pharmaceutically acceptable salt thereof is administered according to a method as follows: continuous oral administration at a dose of 6 mg per time, once daily. Preferably, the pharmaceutically acceptable salt of the compound A is the compound B. Further preferably, the compound B is given in the form of an oral solid formulation containing the compound B. Further preferably, the dose is calculated based on the mass of the compound B.

In some embodiments of the first aspect of the present application, the compound A or the pharmaceutically acceptable salt thereof is administered according to a method as follows: 3-week continuous oral administration at a dose of 6 mg per time, once daily, followed by 1-week interruption. Preferably, the pharmaceutically acceptable salt of the compound A is the compound B. Further preferably, the compound B is given in the form of an oral solid formulation containing the compound B. Further preferably, the dose is calculated based on the mass of the compound B.

In some embodiments of the first aspect of the present application, m is selected from 1, 2, and 3. In some embodiments of the first aspect of the present application, m is 1. In some embodiments of the first aspect of the present application, m is 2. In some embodiments of the first aspect of the present application, m is 3.

In some embodiments of the first aspect of the present application, n is 1, 2, 3, 4, 5, 6, 7, 8, or 9. In some embodiments of the first aspect of the present application, n is 1. In some embodiments of the first aspect of the present application, n is 2. In some embodiments of the first aspect of the present application, n is 3. In some embodiments of the first aspect of the present application, n is 4. In some embodiments of the first aspect of the present application, n is 5. In some embodiments of the first aspect of the present application, n is 6. In some embodiments of the first aspect of the present application, n is 7. In some embodiments of the first aspect of the present application, n is 8. In some embodiments of the first aspect of the present application, n is 9.

In some embodiments of the first aspect of the present application, the compound A or the pharmaceutically acceptable salt thereof is used in combination with one, two, three, or more of drugs commonly used clinically for treating esophageal cancer or carcinoma at the esophagogastric junction in the manufacture of the medicament.

In some embodiments of the first aspect of the present application, the medicament is further used in combination with one, two, three, or more of drugs commonly used clinically for treating esophageal cancer or carcinoma at the esophagogastric junction.

In a second aspect, the present application provides a method for treating a tumor in an individual, comprising administering to the individual a therapeutically effective amount of the compound A or the pharmaceutically acceptable salt thereof according to the first aspect, wherein the tumor is esophageal cancer or carcinoma at the esophagogastric junction.

In some embodiments of the second aspect of the present application, the method further comprises a therapeutically effective amount of an additional drug, wherein the additional drug is selected from one, two, three, or more of drugs commonly used clinically for treating esophageal cancer and carcinoma at the esophagogastric junction.

In the absence of contradictions and conflicts, the technical solutions or technical features according to the first aspect are applicable to the second aspect.

In an aspect 2.1, the present application provides the compound A or the pharmaceutically acceptable salt thereof for use in the treatment of a tumor, wherein the tumor is esophageal cancer or carcinoma at the esophagogastric junction.

In some embodiments of aspect 2.1 of the present application, an additional drug is further included, wherein the additional drug is selected from one, two, three, or more of drugs commonly used clinically for treating esophageal cancer and carcinoma at the esophagogastric junction.

In the absence of contradictions and conflicts, the technical solutions or technical features according to the first aspect are applicable to aspect 2.1.

In a third aspect, the present application provides a pharmaceutical composition for treating a tumor, comprising the compound A or the pharmaceutically acceptable salt thereof according to the first aspect, and optionally a pharmaceutically acceptable carrier, wherein the tumor is esophageal cancer or carcinoma at the esophagogastric junction.

In some embodiments of the third aspect of the present application, the pharmaceutical composition comprises the pharmaceutically acceptable salt of the compound A, and optionally a pharmaceutically acceptable carrier. In some embodiments of the third aspect of the present application, the pharmaceutical composition comprises a hydrochloride of compound A, and optionally a pharmaceutically acceptable carrier. In some embodiments of the third aspect of the present application, the pharmaceutical composition comprises the compound B, and optionally a pharmaceutically acceptable carrier. In some embodiments of the third aspect of the present application, the pharmaceutical composition comprises a solid form of the compound B, and optionally a pharmaceutically acceptable carrier. In some embodiments of the third aspect of the present application, the pharmaceutical composition comprises a crystalline form of the compound B, and optionally a pharmaceutically acceptable carrier. In some embodiments of the third aspect of the present application, the pharmaceutical composition comprises a crystalline form I of the compound B, and optionally a pharmaceutically acceptable carrier.

In some embodiments of the third aspect of the present application, the pharmaceutical composition comprises the compound A or the pharmaceutically acceptable salt thereof and the pharmaceutically acceptable carrier, and further comprises a therapeutically effective amount of an additional drug, wherein the additional drug is selected from drugs commonly used clinically for treating esophageal cancer and carcinoma at the esophagogastric junction.

In some embodiments of the third aspect of the present application, the pharmaceutical composition comprises the compound B and the pharmaceutically acceptable carrier, and further comprises a therapeutically effective amount of an additional drug, wherein the additional drug is selected from one, two, three, or more of drugs commonly used clinically for treating esophageal cancer and carcinoma at the esophagogastric junction.

In the absence of contradictions and conflicts, the technical solutions or technical features according to the first aspect are applicable to the third aspect.

In a fourth aspect, the present application provides a pharmaceutical kit, comprising (a) at least one unit dose of the pharmaceutical composition of the compound A or the pharmaceutically acceptable salt thereof and (b) instructions for use in treating a tumor, wherein the tumor is esophageal cancer or carcinoma at the esophagogastric junction. In some embodiments, provided is a kit, comprising (a) at least one unit dose of an oral formulation of the compound A or the pharmaceutically acceptable salt thereof and (b) instructions for use in treating a tumor, wherein the tumor is esophageal cancer or carcinoma at the esophagogastric junction. The term "unit dose" refers to a pharmaceutical composition or oral formulation packaged in a single package for ease of administration, for example, each portion of the pharmaceutical composition, each tablet, or each capsule.

In the absence of contradictions and conflicts, the technical solutions or technical features according to the first aspect are applicable to the fourth aspect.

In a fifth aspect, the present application provides use of compound A or the pharmaceutically acceptable salt thereof in the manufacture of a medicament for treating a tumor, wherein the tumor is a digestive tract squamous cell carcinoma,

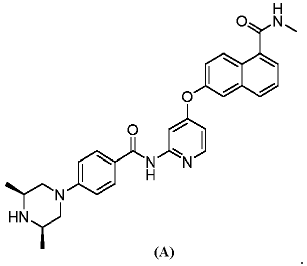

In some embodiments of the fifth aspect of the present application, the tumor is an upper digestive tract squamous cell carcinoma; preferably, the upper digestive tract squamous cell carcinoma is selected from esophageal squamous cell carcinoma and squamous cell carcinoma at the esophagogastric junction.

In some embodiments of the fifth aspect of the present application, the tumor is a tumor with a genetic abnormality or abnormal protein expression of one, two, or three of FGFR, VEGFR, and colony-stimulating factor 1 receptor (CSF1R). Preferably, the tumor is a tumor with a gene mutation in one, two, or three of FGFR, VEGFR, and CSF1R. In some embodiments of the fifth aspect of the present application, the tumor is a locally advanced tumor or a metastatic tumor, preferably an unresectable locally advanced tumor or an unresectable metastatic tumor.

In some embodiments of the fifth aspect of the present application, the tumor is a tumor that has failed a prior treatment.

In some embodiments of the fifth aspect of the present application, the tumor is a tumor that has failed a systemic drug treatment.

In some embodiments of the fifth aspect of the present application, the tumor is a tumor that has failed a first-line treatment.

In some embodiments of the fifth aspect of the present application, the tumor is a tumor that has failed a second-line treatment.

In some embodiments of the fifth aspect of the present application, the tumor is a tumor that has failed a later-line treatment.

In some embodiments of the fifth aspect of the present application, the tumor is a tumor that has failed treatment with one, two, three, or more of an immunotherapeutic drug, a chemotherapeutic drug, radiotherapy, biological therapy, traditional Chinese medicine therapy, and a small molecule targeted drug. In some embodiments of the fifth aspect of the present application, the tumor is a tumor that has failed treatment with radiotherapy, an immunotherapeutic drug, or a chemotherapeutic drug. In some embodiments of the fifth aspect of the present application, the tumor is a tumor that has failed treatment with radiotherapy and an immunotherapeutic drug. In some embodiments of the fifth aspect of the present application, the tumor is a tumor that has failed treatment with radiotherapy and a chemotherapeutic drug. In some embodiments of the fifth aspect of the present application, the tumor is a tumor that has failed treatment with a chemotherapeutic drug and an immunotherapeutic drug. In some embodiments of the fifth aspect of the present application, the tumor is a tumor that has failed treatment with a chemotherapeutic drug, an immunotherapeutic drug, and radiotherapy.

In some embodiments of the fifth aspect of the present application, the aforementioned chemotherapeutic drug is selected from a cytotoxic drug, an antimetabolite drug, an antibiotic drug, an alkaloid drug, a DNA topoisomerase inhibitor chemotherapeutic drug, a microtubule-targeting chemotherapeutic drug, a platinum-based chemotherapeutic drug, and a hormone drug; preferably, the aforementioned chemotherapeutic drug is selected from an antimetabolite drug, an alkaloid drug, a DNA topoisomerase inhibitor chemotherapeutic drug, a microtubule-targeting chemotherapeutic drug, and a platinum-based chemotherapeutic drug.

In some embodiments of the fifth aspect of the present application, the aforementioned chemotherapeutic drug is selected from a taxane-based drug, a platinum-based drug, a fluorouracil-based drug, and a DNA topoisomerase inhibitor chemotherapeutic drug. In some embodiments of the fifth aspect of the present application, the taxane-based drug is selected from paclitaxel, docetaxel, and cabazitaxel, preferably paclitaxel. In some embodiments of the fifth aspect of the present application, the DNA topoisomerase inhibitor chemotherapeutic drug is selected from hydroxycamptothecin, irinotecan, topotecan, etoposide, and teniposide. In some embodiments of the fifth aspect of the present application, the platinum-based drug is selected from cisplatin, carboplatin, sulfatodiamino cyclohexane platinum, lobaplatin, oxaliplatin, and nedaplatin, preferably cisplatin, oxaliplatin, or nedaplatin. In some embodiments of the fifth aspect of the present application, the aforementioned chemotherapeutic drug is selected from paclitaxel, cisplatin, and nedaplatin.

In some embodiments of the fifth aspect of the present application, the aforementioned immunotherapeutic drug is selected from an anti-programmed death receptor 1 antibody (anti-PD-1 antibody), an anti-programmed death receptor-ligand 1 antibody (anti-PD-L1 antibody), and an antibody-drug conjugate. Preferably, the antibody-drug conjugate is an anti-HER2 antibody-drug conjugate. In some embodiments of the fifth aspect of the present application, the anti-PD-1 antibody is selected from pembrolizumab, camrelizumab, tislelizumab, nivolumab, cemiplimab, sintilimab, and toripalimab; preferably, the anti-PD-1 antibody is selected from toripalimab, sintilimab, and tislelizumab. In some embodiments of the fifth aspect of the present application, the anti-PD-L1 antibody is selected from atezolizumab, avelumab, and durvalumab. In some embodiments of the fifth aspect of the present application, the anti-HER2 antibody-drug conjugate is selected from trastuzumab and disitamab.

In some embodiments of the fifth aspect of the present application, the tumor is a tumor that has failed treatment with a chemotherapeutic drug and an immunotherapeutic drug; preferably, the tumor that has failed treatment is a tumor that has failed treatment with a taxane-based chemotherapeutic drug + a platinum-based chemotherapeutic drug + an anti-PD-1 antibody; preferably, the tumor that has failed treatment is a tumor that has failed treatment with one, two, three, or more of toripalimab + paclitaxel + cisplatin, sintilimab + paclitaxel + cisplatin, paclitaxel + lobaplatin + camrelizumab, paclitaxel + nedaplatin, and sintilimab. Preferably, the tumor is esophageal squamous cell carcinoma.

In some embodiments of the fifth aspect of the present application, the tumor is a tumor that has failed treatment with a chemotherapeutic drug; preferably, the tumor is a tumor that has failed treatment with a taxane-based chemotherapeutic drug + a platinum-based chemotherapeutic drug; preferably, the tumor that has failed treatment is a tumor that has failed treatment with paclitaxel + cisplatin or paclitaxel + nedaplatin. Preferably, the tumor is esophageal squamous cell carcinoma.

In some embodiments of the fifth aspect of the present application, the tumor is a tumor that has failed treatment with a chemotherapeutic drug, immunotherapy, and radiotherapy; preferably, the tumor is a tumor that has failed treatment with a taxane-based chemotherapeutic drug + a platinum-based chemotherapeutic drug + radiotherapy + an anti-PD-1 antibody; preferably, the tumor that has failed treatment is a tumor that has failed treatment with tislelizumab + radiotherapy + chemotherapy. Preferably, the tumor is esophageal squamous cell carcinoma.

In the absence of contradictions and conflicts, the technical solutions or technical features according to the first aspect are applicable to the fifth aspect.

In a sixth aspect, the present application provides use of the compound A or the pharmaceutically acceptable salt thereof in the manufacture of a medicament for treating a tumor, 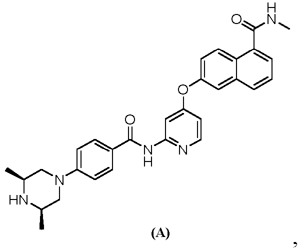 wherein the tumor is squamous cell carcinoma.

In some embodiments of the sixth aspect of the present application, the tumor is selected from head and neck squamous cell carcinoma, digestive tract squamous cell carcinoma, and respiratory tract squamous cell carcinoma; preferably, the tumor is selected from head and neck squamous cell carcinoma, upper digestive tract squamous cell carcinoma, and respiratory tract squamous cell carcinoma; further preferably, the tumor is selected from esophageal squamous cell carcinoma, squamous cell carcinoma at the esophagogastric junction, head and neck squamous cell carcinoma, and pulmonary squamous cell carcinoma; further preferably, the tumor is selected from esophageal squamous cell carcinoma, squamous cell carcinoma at the esophagogastric junction, nasopharyngeal squamous cell carcinoma, and pulmonary squamous cell carcinoma; further preferably, the tumor is selected from nasopharyngeal squamous cell carcinoma, esophageal squamous cell carcinoma, and pulmonary squamous cell carcinoma; further preferably, the tumor is esophageal squamous cell carcinoma.

In some embodiments of the sixth aspect of the present application, the tumor is a tumor with a genetic abnormality or abnormal protein expression of one, two, or three of FGFR, VEGFR, and CSF1R. Preferably, the tumor is a tumor with a genetic abnormality of one, two, or three of FGFR, VEGFR, and CSF1R.

In some embodiments of the sixth aspect of the present application, the tumor is a locally advanced tumor or a metastatic tumor, preferably an unresectable locally advanced tumor or an unresectable metastatic tumor.

In some embodiments of the sixth aspect of the present application, the tumor is a tumor that has failed a prior treatment or has relapsed.

In some embodiments of the sixth aspect of the present application, the tumor is a tumor that has failed a systemic drug treatment.

In some embodiments of the sixth aspect of the present application, the tumor is a tumor that has failed a first-line treatment.

In some embodiments of the sixth aspect of the present application, the tumor is a tumor that has failed a second-line treatment or a later-line treatment.

In some embodiments of the sixth aspect of the present application, the tumor is a tumor that has failed treatment with one, two, three, or more of an immunotherapeutic drug, a chemotherapeutic drug, radiotherapy, biological therapy, traditional Chinese medicine therapy, and a small molecule targeted drug. In some embodiments of the sixth aspect of the present application, the tumor is a tumor that has failed treatment with radiotherapy, an immunotherapeutic drug, or a chemotherapeutic drug. In some embodiments of the sixth aspect of the present application, the tumor is a tumor that has failed treatment with radiotherapy and an immunotherapeutic drug. In some embodiments of the sixth aspect of the present application, the tumor is a tumor that has failed treatment with radiotherapy and a chemotherapeutic drug. In some embodiments of the sixth aspect of the present application, the tumor is a tumor that has failed treatment with a chemotherapeutic drug and an immunotherapeutic drug. In some embodiments of the sixth aspect of the present application, the tumor is a tumor that has failed treatment with a chemotherapeutic drug, an immunotherapeutic drug, and radiotherapy.

In some embodiments of the sixth aspect of the present application, the aforementioned chemotherapeutic drug is selected from a cytotoxic drug, an antimetabolite drug, an antibiotic drug, an alkaloid drug, a DNA topoisomerase inhibitor chemotherapeutic drug, a microtubule-targeting chemotherapeutic drug, a platinum-based chemotherapeutic drug, and a hormone drug; preferably, the aforementioned chemotherapeutic drug is selected from an antimetabolite drug, an alkaloid drug, a DNA topoisomerase inhibitor chemotherapeutic drug, a microtubule-targeting chemotherapeutic drug, and a platinum-based chemotherapeutic drug.

In some embodiments of the sixth aspect of the present application, the aforementioned chemotherapeutic drug is selected from a taxane-based drug, a platinum-based drug, a fluorouracil-based drug, and a DNA topoisomerase inhibitor chemotherapeutic drug. In some embodiments, the taxane-based drug is selected from paclitaxel, docetaxel, and cabazitaxel, preferably paclitaxel. In some embodiments, the DNA topoisomerase inhibitor chemotherapeutic drug is selected from hydroxycamptothecin, irinotecan, topotecan, etoposide, and teniposide. In some embodiments, the platinum-based drug is selected from cisplatin, carboplatin, sulfatodiamino cyclohexane platinum, lobaplatin, oxaliplatin, and nedaplatin, preferably cisplatin, oxaliplatin, or nedaplatin. In some embodiments, the aforementioned chemotherapeutic drug is selected from paclitaxel, cisplatin, and nedaplatin.

In some embodiments of the sixth aspect of the present application, the aforementioned immunotherapeutic drug is selected from an anti-programmed death receptor 1 antibody (anti-PD-1 antibody), an anti-programmed death receptor-ligand 1 antibody (anti-PD-L1 antibody), and an antibody-drug conjugate. Preferably, the antibody-drug conjugate is an anti-HER2 antibody-drug conjugate. In some embodiments, the anti-PD-1 antibody is selected from pembrolizumab, camrelizumab, tislelizumab, nivolumab, cemiplimab, sintilimab, and toripalimab; preferably, the anti-PD-1 antibody is selected from toripalimab, sintilimab, and tislelizumab. In some embodiments, the anti-PD-L1 antibody is selected from atezolizumab, avelumab, and durvalumab. In some embodiments, the anti-HER2 antibody-drug conjugate is selected from trastuzumab and disitamab.

In some embodiments of the sixth aspect of the present application, the tumor is a tumor that has failed treatment with a chemotherapeutic drug and an immunotherapeutic drug; preferably, the tumor is a tumor that has failed treatment with a taxane-based chemotherapeutic drug + a platinum-based chemotherapeutic drug + an anti-PD-1 antibody; preferably, the tumor is a tumor that has failed treatment with one, two, three, or more of toripalimab + paclitaxel + cisplatin, sintilimab + paclitaxel + cisplatin, paclitaxel + lobaplatin + camrelizumab, paclitaxel + nedaplatin, and sintilimab.

In some embodiments of the sixth aspect of the present application, the tumor is a tumor that has failed treatment with a chemotherapeutic drug; preferably, the tumor is a tumor that has failed treatment with a taxane-based chemotherapeutic drug + a platinum-based chemotherapeutic drug; preferably, the tumor is a tumor that has failed treatment with paclitaxel + cisplatin, paclitaxel + carboplatin, or paclitaxel + nedaplatin.

In some embodiments of the sixth aspect of the present application, the tumor is a tumor that has failed treatment with a chemotherapeutic drug, immunotherapy, and radiotherapy; preferably, the tumor is a tumor that has failed treatment with a taxane-based chemotherapeutic drug + a platinum-based chemotherapeutic drug + radiotherapy + an anti-PD-1 antibody; preferably, the tumor is a tumor that has failed treatment with tislelizumab + radiotherapy + chemotherapy.

In the absence of contradictions and conflicts, the technical solutions or technical features according to the first aspect are applicable to the sixth aspect.

In a seventh aspect, the present application provides a method for treating a tumor in an individual, comprising administering to the individual a therapeutically effective amount of the compound A or the pharmaceutically acceptable salt thereof according to the first aspect, wherein the tumor is a squamous cell carcinoma.

In some embodiments of the seventh aspect of the present application, the method further comprises a therapeutically effective amount of an additional drug, wherein the additional drug is selected from one, two, three, or more of drugs commonly used clinically for treating squamous cell carcinoma.

In the absence of contradictions and conflicts, the technical solutions or technical features according to the first aspect or the sixth aspect are applicable to the seventh aspect.

In an eighth aspect, the present application provides the compound A or the pharmaceutically acceptable salt thereof for use in the treatment of a tumor, wherein the tumor is a squamous cell carcinoma.

In some embodiments of the eighth aspect of the present application, an additional drug is further included, wherein the additional drug is selected from one, two, three, or more of drugs commonly used clinically for treating squamous cell carcinoma.

In the absence of contradictions and conflicts, the technical solutions or technical features according to the first aspect or the sixth aspect are applicable to the eighth aspect.

In a ninth aspect, the present application provides a pharmaceutical composition for treating a tumor, comprising the compound A or the pharmaceutically acceptable salt thereof, and optionally a pharmaceutically acceptable carrier, wherein the tumor is a squamous cell carcinoma.

In some embodiments of the ninth aspect of the present application, the pharmaceutical composition comprises the pharmaceutically acceptable salt of the compound A, and optionally a pharmaceutically acceptable carrier. In some embodiments of the ninth aspect of the present application, the pharmaceutical composition comprises a hydrochloride of compound A, and optionally a pharmaceutically acceptable carrier. In some embodiments of the ninth aspect of the present application, the pharmaceutical composition comprises the compound B, and optionally a pharmaceutically acceptable carrier. In some embodiments of the ninth aspect of the present application, the pharmaceutical composition comprises a solid form of the compound B, and optionally a pharmaceutically acceptable carrier. In some embodiments of the ninth aspect of the present application, the pharmaceutical composition comprises a crystalline form of the compound B, and optionally a pharmaceutically acceptable carrier. In some embodiments of the ninth aspect of the present application, the pharmaceutical composition comprises a crystalline form I of the compound B, and optionally a pharmaceutically acceptable carrier.

In some embodiments of the ninth aspect of the present application, the pharmaceutical composition comprises the compound A or the pharmaceutically acceptable salt thereof and the pharmaceutically acceptable carrier, and further comprises a therapeutically effective amount of an additional drug, wherein the additional drug is selected from drugs commonly used clinically for treating squamous cell carcinoma.

In some embodiments of the ninth aspect of the present application, the pharmaceutical composition comprises the compound B and the pharmaceutically acceptable carrier, and further comprises a therapeutically effective amount of an additional drug, wherein the additional drug is selected from one, two, three, or more of drugs commonly used clinically for treating squamous cell carcinoma.

In the absence of contradictions and conflicts, the technical solutions or technical features according to the first aspect or the sixth aspect are applicable to the ninth aspect.

In some embodiments, in the provided use (first aspect), method (second aspect), compound or pharmaceutically acceptable salt thereof (aspect 2.1), pharmaceutical composition (third aspect), kit (fourth aspect), use (fifth aspect), use (sixth aspect), method (seventh aspect), compound or pharmaceutically acceptable salt thereof (eighth aspect) or pharmaceutical composition (ninth aspect), the compound A or the pharmaceutically acceptable salt thereof is administered alone to a patient as the sole active ingredient. In some embodiments, in the provided use (first aspect), method (second aspect), compound or pharmaceutically acceptable salt thereof (aspect 2.1), pharmaceutical composition (third aspect), kit (fourth aspect), use (fifth aspect), method (seventh aspect), compound or pharmaceutically acceptable salt thereof (eighth aspect) or pharmaceutical composition (ninth aspect), an additional drug is further included, and the compound A or the pharmaceutically acceptable salt thereof is administered to a patient with a tumor simultaneously with or sequentially to the additional drug, wherein the tumor is the esophageal cancer or carcinoma at the esophagogastric junction according to the first aspect, the digestive tract squamous cell carcinoma according to the fifth aspect, or the squamous cell carcinoma according to the sixth aspect. Preferably, the additional drug includes, but is not limited to, a chemotherapeutic drug, an immunotherapeutic drug, and a small molecule targeted drug.

The "prior treatment" described in the present application includes, but is not limited to, one, two, three, or more of radiotherapy, chemotherapy, and targeted therapy (including immunotherapy and small molecule targeted drug therapy).

The "immunotherapy" described in the present application includes immune checkpoint inhibitors, tumor vaccines, cellular immunotherapy, non-specific immunomodulators, and immunomodulatory antibody-drug conjugates (or referred to as antibody-drug conjugates).

The "first-line treatment" described in the present application is not limited to first-line drug treatment regimens approved for the treatment of specific tumor conditions (e.g., esophageal cancer, carcinoma at the esophagogastric junction, nasopharyngeal squamous cell carcinoma, pulmonary squamous cell carcinoma, etc.), but also includes non-first-line chemotherapy regimens, immunotherapy, targeted therapy, and other systemic drug treatments used as the initial treatment of specific tumor conditions after diagnosis; the "failed a first-line treatment" also includes the occurrence of progressive disease within 6 months of adjuvant chemotherapy.

The "administered alone" described in the present application means that a drug does not need to be used in combination with other anti-tumor drugs, but the use of some auxiliary drugs without anti-tumor effects is not excluded.

The present application has achieved the following beneficial technical effects: (1) the compound A or the pharmaceutically acceptable salt thereof (e.g., compound B) exhibits clinical benefits for esophageal cancer, carcinoma at the esophagogastric junction, and/or squamous cell carcinoma (e.g., nasopharyngeal squamous cell carcinoma, pulmonary squamous cell carcinoma, and esophageal squamous cell carcinoma), and therefore has clinical potential for the treatment of esophageal cancer, carcinoma at the esophagogastric junction, and squamous cell carcinoma; (2) the compound A or the pharmaceutically acceptable salt thereof (e.g., compound B) exhibits relatively good tolerability in phase I clinical trials, and has fewer types of adverse effects, suggesting a relatively good clinical safety.

### Definitions and Description

Unless otherwise stated, the following terms and phrases used herein are intended to have the following meanings. A particular phrase or term, unless otherwise specifically defined, should not be considered as uncertain or unclear, but should be construed according to its common meaning.

The "pharmaceutically acceptable" mentioned in the present application refers to that when a substance is used for preparing a pharmaceutical composition, the pharmaceutical composition is generally safe, non-toxic, and desirable biologically and otherwise, and inclusion of the substance is acceptable for pharmaceutical use in humans.

The "solid form" mentioned in the present application refers to a compound in a solid state, including a crystalline form and an amorphous form of the compound B.

The "crystalline form" mentioned in the present application refers to a compound in a crystalline state, including a water-free and solvent-free form (or referred to as an anhydrous form), a hydrate form, a solvate form, and a cocrystal form of compound B. The crystalline form is preferably a water-free and solvent-free form or a hydrate form, further preferably a water-free and solvent-free form.

The "treat" or its variant mentioned in the present application refers to alleviating, relieving, or ameliorating a symptom of a disease or condition, ameliorating an underlying metabolism-induced symptom, or inhibiting a disease or symptom, such as arresting the progression of a disease or condition, relieving a disease or condition, causing regression of a disease or condition, relieving a disorder caused by a disease or condition, or arresting a symptom of a disease or condition.

The "failed treatment" or its variant mentioned in the present application refers to progressive disease during treatment, relapse after treatment, or intolerance to toxic and side effects.

The "systemic drug treatment" mentioned in the present application includes neoadjuvant therapy and adjuvant therapy, as well as chemotherapy, small molecule targeted therapy, and immunotherapy for patients with advanced diseases.

The "genetic abnormality" mentioned in the present application refers to one or more of the events of fusion, amplification, rearrangement, or mutation of the genes of FGFR, VEGFR, or CSF1R. The "abnormal protein expression" mentioned in the present application refers to one or more of the events of protein overexpression, misfolding, or kinase domain duplication.

The "optional" or "optionally" mentioned in the present application refers to inclusion or exclusion, meaning that the subsequently described event or circumstance may, but not necessarily, occur, and the description includes instances where the event or circumstance occurs or does not occur.

The "more" or "more times" mentioned in the present application, in some cases, refers to 4 or more, for example, the case of "one, two, three or more"; in some cases, it refers to 2, 3, or more, for example, the case of "one or more".

The "junction" of "carcinoma at the esophagogastric junction" in the present application is also referred to as "connection", thus the "carcinoma at the esophagogastric junction" is synonymous with "carcinoma at the esophagogastric connection".

The "head and neck squamous carcinoma" in the present application is also referred to as "head and neck squamous cell carcinoma". The "digestive tract squamous carcinoma" in the present application is also referred to as "digestive tract squamous cell carcinoma". The "respiratory tract squamous carcinoma" in the present application is also referred to as "respiratory tract squamous cell carcinoma". The "upper digestive tract squamous carcinoma" in the present application is also referred to as "upper digestive tract squamous cell carcinoma". The "esophageal squamous carcinoma" in the present application is also referred to as "esophageal squamous cell carcinoma" or "esophagus squamous carcinoma" or "esophagus squamous cell carcinoma". The "squamous carcinoma at the esophagogastric junction" in the present application is also referred to as "squamous cell carcinoma at the esophagogastric junction". The "pulmonary squamous carcinoma" in the present application is also referred to as "pulmonary squamous cell carcinoma". The "nasopharyngeal squamous carcinoma" in the present application is also referred to as "nasopharyngeal squamous cell carcinoma" or "nasopharynx squamous cell carcinoma".

The pharmaceutical composition of the present application can be prepared using methods known in the art.

The term "pharmaceutical composition" refers to a mixture consisting of one or more of the compounds or the pharmaceutically acceptable salts thereof of the present application and a pharmaceutically acceptable carrier. The purpose of the pharmaceutical composition is to facilitate the administration of the pharmaceutically acceptable salt, the stereoisomer, or the prodrug of the compound of the present application to an organism. The pharmaceutical composition of the present application can be prepared using a conventional method in the art.

In the context of the present application, the term "pharmaceutically acceptable carrier" can also be referred to as "excipient" or "pharmaceutically acceptable auxiliary material", which refers to those auxiliary materials that do not have a significant irritating effect on an organism and do not impair the bioactivity and performance of the active compound. The term "pharmaceutically acceptable carrier" includes, but is not limited to: solvents, propellants, solubilizers, cosolvents, emulsifiers, colorants, binders, fillers, etc. Those skilled in the art can select a specific pharmaceutically acceptable auxiliary material according to the actual need to prepare a desired dosage form, for example, a tablet. Knowledge about auxiliary materials is well-known to those skilled in the art, and reference can be made, for example, to Pharmaceutics (edited by Fu-De Cui, 5th edition, People's Medical Publishing House, 2003).

ORR: the percentage of subjects with complete remission (CR) and partial remission (PR) among all enrolled subjects from the date of the first dose.

PFS: the duration from the date of the first dose to the date of tumor progression (evaluated by diagnostic imaging as PD) or to the date of all-cause death (whichever occurs first).

OS: the time from the date of the first dose to the date of death from any cause (for those subjects who are lost to follow-up, the last follow-up date; for those subjects still alive at the end of the study, the follow-up end date). DCR: defined as the percentage of subjects with CR, PR, or stable disease (SD) among all enrolled subjects from the date of the first dose.

DOR: the time from the first evaluation of the tumor as CR or PR to the first evaluation of the tumor as PD or death from any cause.

CR: refers to complete remission, meaning that all target lesions have disappeared, and the short diameter of all pathological lymph nodes (including target nodes and non-target nodes) must be reduced to < 10 mm.

PR: refers to partial remission, meaning that the sum of the diameters of target lesions has reduced by at least 30% compared to the baseline level.

PD: refers to progressive disease, meaning that the sum of the measured diameters of all target lesions has increased by at least 20% with reference to the minimum value measured throughout the entire study (or with reference to the baseline value if it is measured to be minimum), and the absolute increase in the sum of the diameters must be at least 5 mm; the emergence of one or more new lesions is also considered as progressive disease.

SD: refers to stable disease, meaning that the target lesions are neither sufficiently reduced to level of PR nor sufficiently increased to level of PD, but somewhere in between.

The words "include", "contain", "comprise" or its variant should be understood in an open, non-exclusive sense, i.e., "including but not limited to".

In the scope of the present application, various options of any feature can be combined with various options of other features to form a plurality of different embodiments. The present application is intended to include all possible embodiments consisting of various options of all technical features. It is to be understood that within the scope of the present disclosure, the above various technical features of the present disclosure and the technical features specifically described hereinafter (as in the examples) may be combined with each other to form new or preferred technical solutions, which will not be elaborated one by one due to space limitations.

The compounds of the present application can be prepared by a variety of synthetic methods known to those skilled in the art, including the specific embodiments listed below, embodiments formed by combinations thereof with other chemical synthetic methods, and equivalent substitutions thereof known to those skilled in the art. Preferred embodiments include, but are not limited to, the examples of the present disclosure. For brevity, details are omitted here.

The chemical reactions of the specific embodiments of the present application are carried out in a proper solvent that must be suitable for the chemical changes in the present application and the reagents and materials required. In order to obtain the compounds of the present application, it is sometimes necessary for those skilled in the art to modify or select a synthesis procedure or a reaction process based on the existing embodiments.

The present application is described in detail below through examples; however, these examples are not intended to limit the present application in any way.

All reagents and solvents used in the present application are commercially available and can be used without further purification.

### DETAILED DESCRIPTION

The technical solutions of the present application will be further described in detail with reference to the following specific examples, test examples, and experimental examples. The following examples, test examples, and experimental examples are merely exemplary illustrations and explanations of the present disclosure, and should not be construed as limiting the protection scope of the present disclosure. Non-essential improvements and modifications to the embodiments made by those skilled in the art in accordance with the above content disclosed herein shall still fall within the protection scope of the present disclosure. Unless otherwise stated, the starting materials and reagents (e.g., organic solvents, inorganic solvents, etc.) used in the following examples and test examples are all commercially available products, or can be prepared or formulated by known methods or reagent specifications.

In the following examples, the analysis and detection conditions are as follows:
1. X-ray powder diffractometer (XRPD)
Instrument: BRUKER D8 Advance X-ray powder diffractometer, Germany.
Conditions: Cu-Kα radiation (λ = 1.5418 Å), tube voltage: 40 kV, tube current: 40 mA, 20 scanning range: 3-40°, scanning step size: 0.02° (2θ), scanning speed: 10 s/step, sample tray: a zero background sample tray.
Method: a sample was spread on a zero background monocrystalline silicon sample tray and was gently pressed using a medicine spoon to be flat for measurement.

2. Chloride
Detection instrument: Dionex ICS-900 ion chromatograph
Column: Dionex Ion Pac AS11-HC anion chromatography column (specification: 4 × 250 mm)
Determination method: 10 µL of each of the control solution and the sample solution was measured precisely and injected into the ion chromatograph, the chromatogram was recorded, and the chloride ion content was calculated based on peak area according to the external standard method.

3. Dynamic vapor sorption/desorption (DVS) analysis
Instrument: DVS Intrinsic Plus (SMS, UK).
Method: a sample was placed in a tared sample basket, the weight of the sample was automatically measured, and the sample was analyzed according to the parameters in the table below.

| Instrument information | SMS, DVS Intrinsic PLUS |
|---|---|
| dm/dt | 0.002%/min |
| Drying/testing temperature | 40 °C/25 °C |
| Cycle | Full cycle |
| dm/dt minimum equilibration time | 30 min |
| dm/dt maximum equilibration time | 120 min |
| Data storage rate | 5 s |
| Gas and flow rate | N₂, 200 sccm |
| Post-run flow rate | 200 sccm |
| Step size | 10% RH |
| Method | Sorption: 0, 10, 20, 30, 40, 50, 60, 70, 80, 90 |
| | Desorption: 80, 70, 60, 50, 40, 30, 20, 10, 0 |

4. Detection conditions for thermogravimetric analysis (TGA)
Instrument: Discovery TGA 55.
Conditions: temperature range: room temperature to 350 °C; heating rate: 10 °C/min; purging gas: nitrogen; flow rate in equilibration chamber: 40 mL/min; flow rate in sample chamber: 25 mL/min.
Sample: 1-5 mg.

### Example 1: Preparation of Compound B

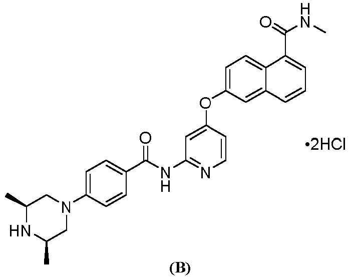

Compound A (0.52 g, prepared according to Preparation Example 10 of WO2017140269A1) and methanol (25 mL) were added to a reaction flask and stirred, and after the system was dispersed uniformly, a solution of hydrochloric acid in methanol (1.2 mL, 2 mol/L) was added dropwise under stirring. After the dropwise addition was completed, the mixture was successively stirred for 5 h. Then, the mixture was filtered and dried under vacuum (dried under vacuum at 30 °C for 12 h until a constant weight was achieved, and then baked under vacuum at 45 °C until the solvent residue was qualified) to obtain a solid (0.53 g). Nuclear magnetic resonance detection confirmed the formation of the salt.

By measuring the chloride ion content using ion chromatography, the stoichiometric ratio of the hydrochloride was calculated (see the table below), and the base/acid ratio of the hydrochloride was concluded to be 1:2.

**Table 1**

| Name | Theoretical stoichiometric ratio (base/acid) | Theoretical chloride ion content (%) | Measured chloride ion content (%) |
|---|---|---|---|
| Example 1 | 1:2 | 12.2% | 11.7% |

The obtained solid sample was taken and subjected to X-ray powder diffraction, and the results demonstrated that the solid sample had a good crystallinity. It was designated as crystalline form I, with its diffraction peak data shown in Table 2.

**Table 2 XRPD diffraction peak data of crystalline form I sample obtained in Example 1**

| 2θ (°) | Relative peak intensity (%) | 2θ (°) | Relative peak intensity (%) | 2θ (°) | Relative peak intensity (%) |
|---|---|---|---|---|---|
| 4.964 | 100.0 | 18.012 | 7.8 | 23.857 | 13.0 |
| 9.960 | 7.4 | 19.318 | 24.9 | 24.246 | 20.4 |
| 10.371 | 17.1 | 20.048 | 8.5 | 25.179 | 55.9 |
| 11.192 | 14.0 | 20.460 | 43.5 | 26.555 | 12.4 |
| 13.485 | 93.9 | 21.394 | 97.3 | 28.539 | 14.8 |
| 14.015 | 11.0 | 21.943 | 10.6 | 30.222 | 9.3 |
| 15.704 | 5.5 | 22.460 | 20.3 | 31.974 | 5.8 |
| 17.326 | 4.3 | 23.340 | 22.2 | 33.014 | 6.0 |

### Example 2: Preparation of Crystalline Form I of Compound B

Compound A (1.0432 g, prepared according to Preparation Example 10 of WO2017140269A1) and purified water (3 mL) were added to a reaction flask and stirred until the system was dispersed uniformly. An aqueous hydrochloric acid solution (2 N) was added dropwise, and the mixture was stirred until complete dissolution. The crystalline form I sample (0.01 g) obtained in Example 1 was added as a crystal seed, and the resulting mixture was stirred for crystallization for 20-24 h and then filtered under vacuum. The filter cake was dried under vacuum at 30 °C for 12 h and then baked under vacuum at 45 °C until the solvent residue was qualified, thus obtaining a solid (1.112 g). The solid was detected, confirming that the crystalline form I of compound B was obtained, with good crystallinity. The XRPD characterization data of the sample obtained are shown in Table 3. The sample was subjected to TGA-DSC test, and the results demonstrated that the sample did not contain water of crystallization and solvent of crystallization.

**Table 3 XRPD diffraction peak data of crystalline form I sample obtained in Example 2**

| 2θ (°) | Relative peak intensity (%) | 2θ (°) | Relative peak intensity (%) | 2θ (°) | Relative peak intensity (%) |
|---|---|---|---|---|---|
| 4.955 | 37.7 | 19.305 | 31.6 | 25.246 | 50.5 |
| 9.982 | 5.20 | 20.075 | 8.5 | 26.572 | 14.9 |
| 10.384 | 13.7 | 20.496 | 41.1 | 28.654 | 8.4 |
| 11.212 | 10.4 | 21.397 | 87.5 | 30.056 | 6.5 |
| 13.503 | 100 | 22.015 | 10.2 | 32.071 | 6.7 |
| 14.056 | 9.9 | 22.526 | 17.7 | 33.032 | 4.8 |
| 15.733 | 7 | 23.367 | 22.8 | | |
| 17.382 | 5.6 | 23.884 | 17 | | |
| 18.029 | 4.4 | 24.298 | 17.7 | | |

### Example 3: Preparation of Other Salts of Compound A

To each of flasks containing 5 mL of a 0.02 mol/mL solution of an acid (phosphoric acid, maleic acid, tartaric acid, and fumaric acid) in tetrahydrofuran/methanol (1:1, volume ratio) was added 10 mL of a 0.01 mol/mL solution of compound A in tetrahydrofuran/methanol (1:1, volume ratio), and the mixtures were stirred homogeneously, followed by reaction at 40 °C for 1 h. The reaction liquids were volatilized and dried at 50 °C for 3-4 h. Solids were obtained in all cases, which were confirmed by nuclear magnetic resonance and ion chromatography to be the phosphate, the maleate, the tartrate, and the fumarate of compound A, respectively.

Further XRPD detection demonstrated that the crystallinity of the obtained solids was good.

### Test Example 1: Solubility Experiment

The solubilities of compound A, the sample obtained in Example 2 (compound B), and the samples obtained in Example 3 were measured in water. Then, the solubility tests of compound A and the sample obtained in Example 2 (compound B) were separately performed in a medium of Na₂HPO₄-citric acid buffer (pH 4.6). The experimental results are shown in Table 4 and Table 5, respectively.

**Table 4 Solubility experimental results of compound A and different salt forms thereof (25 °C, mg/mL)**

| **Solvent/salt** | **Compound A** | **Compound B** | **Phosphate** | **Maleate** | **Tartrate** | **Fumarate** |
|---|---|---|---|---|---|---|
| Deionized water | 0.008 | 16.2 | 3.5 | 2.0 | 1.4 | 1.3 |

**Table 5 Solubility experimental results of compound A and compound B (25 °C, mg/mL)**

| **Solvent/salt** | **Compound A** | **Compound B** |
|---|---|---|
| Na₂HPO₄-citric acid buffer (pH 4.6) | 0.045 | 0.1 |

The results demonstrated that the solubilities of the sample obtained in Example 2 and the samples of different salts obtained in Example 3 in water were good and significantly better than those of free bases thereof. The solubilities meet the general requirements of oral dosage forms for the solubility of the drug substance, and especially, the solubility of the sample obtained in Example 2 meets the general requirements of various pharmaceutical dosage forms (e.g., solid oral dosage forms, injections, oral liquids, etc.) for the solubility of the drug substance. The sample obtained in Example 2 also had a better solubility in the buffer compared to compound A.

### Test Example 2: Water Sorption and Desorption Experiment

The samples obtained in Example 2 and Example 3 were investigated for sorption and desorption of moisture under the relative humidity of 40%-80% at 25 °C using a dynamic vapor sorption (DVS) instrument to determine the hygroscopicity of various salt forms. The experimental results are shown in Table 6.

**Table 6 Hygroscopicity experimental results of different salt forms of compound A (DVS, 40%-80%)**

| **Salt form** | **Sample from Example 2** | **Phosphate** | **Maleate** | **Tartrate** | **Fumarate** |
|---|---|---|---|---|---|
| Hygroscopicity (%) | 0.65 | 3.75 | 0.81 | 2.86 | 2.11 |

The results demonstrated that: the samples obtained in Example 3 were slightly hygroscopic (maleate) or hygroscopic, but the values of the hygroscopic weight gain were not high; the sample obtained in Example 2 was slightly hygroscopic but had a lower hygroscopic weight gain.

### Test Example 3: Solid Stability Experiment

The sample obtained in Example 2 was investigated for physical stability and chemical stability under the conditions of 40 °C/75% RH (open) and 60 °C (sealed) for 7 days, and for physical stability under the conditions of room temperature/92.5% RH (open) for 10 days. The experimental results are shown in Table 7 below.

**Table 7 Solid stability experimental results**

| Parameter/condition | Initial state 0 day | 40 °C/75% RH for 7 days | 60 °C for 7 days | 92.5% RH for 10 days |
|---|---|---|---|---|
| Purity/% | 99.85 | 99.86 | 99.85 | / |
| Crystalline form | Crystalline form I | Not changed | Not changed | Not changed |

| | | | | |
|---|---|---|---|---|
| Note: the "/" indicates undetected; the crystalline form results were obtained using the X-ray powder diffraction method. | | | | |

The results demonstrated that after compound B was left to stand under the conditions of 40 °C/75% RH and 60 °C for 7 days, the purity thereof showed no significant changes, and the crystalline form thereof was not changed; after compound B was left to stand under the condition of 92.5% RH for 10 days, the crystalline form thereof was also not changed. The results indicated that compound B had good chemical stability, and the crystalline form I thereof had good physical stability.

### Test Example 4: Grinding Stability Experiment

The samples obtained in Example 2 were mechanically ground using a mortar for 2 min and 5 min, respectively, and X-ray powder diffraction detection demonstrated that the crystalline form I was not changed after grinding. Furthermore, the samples obtained in Example 2 were mechanically ground after a suitable amount of an organic solvent (methanol, ethanol, acetone, acetonitrile, ethyl acetate, etc.) was separately added dropwise, and X-ray powder diffraction detection demonstrated that the crystalline form I was not changed after grinding.

In conclusion, the salts of compound A in the present application have relatively good pharmaceutical potential, and particularly, the compound B and the crystalline form I thereof have better pharmaceutical potential compared with other crystalline forms or other salt forms.

The preparation, testing, and other related details of compound B and the crystalline form I thereof can also be found in Patent Application No. CN202210268968.7 or PCT/CN2023/082140, the entire contents of which are incorporated herein by reference.

### Experimental Example 1: Phase I Clinical Trial and Results Thereof

Drug: Compound B, tablets.

### 1. Methodology

1.1 Inclusion criteria: The main criteria include: (1) an age of 18-70 years old (including 18 years old and 70 years old) when signing an informed consent form; (2) patients with histopathologically or cytologically confirmed unresectable locally advanced or metastatic solid tumors, who have failed or are intolerant to standard treatments, or have no standard treatment regimens available as options; (3) the presence of at least one measurable lesion meeting RECIST v1.1 definition; (4) ECOG performance status (PS) score: 0-1; and so on.

### 1.2 Administration regimen:

(1) Dose escalation stage: initial doses: 1 mg, 2 mg, 4 mg, 6 mg, 9 mg, 12 mg, 16 mg, 20 mg, and 25 mg, administered orally once daily. After 7-days single administration stage, 28-days multiple administration stage began (continuous administration, with every 28 days constituting one cycle, until progressive disease or intolerable toxic reactions occurred). The subjects who had completed the treatment and observation for the 7-days single administration stage and the first cycle of the 28-days multiple administration stage, and who had acquired clinical benefit and could tolerate the drug treatment, could enter an extended treatment period based on their willingness. In the extended treatment period, every 4 weeks constituted one cycle.
(2) Dose expansion stage: 4 mg and 6 mg, administered orally once daily. For the continuous administration group, every 4 weeks constituted 1 cycle; for the group of 3-week administration and then 1-week interruption, every 4 weeks constituted one cycle; for the group of 2-week administration and then 1-week interruption, every 3 weeks constituted 1 cycle.

1.3 Assessment of results: (1) Effectiveness evaluation: In this study, for the dose escalation stage, efficacy evaluation and data analysis were performed at the 4th week, 8th week and 12th week after the first day of the 28-days multiple administration stage and then every 8 weeks thereafter. For the dose expansion stage, efficacy evaluation on the group of 3-week administration and then 1-week interruption was performed at the 4th week, 8th week and 12th week after the first day of the 28-days multiple administration stage and then every 8 weeks thereafter; efficacy evaluation on the group of 2-week administration and then 1-week interruption was performed every 6 weeks after the first day of the 28-days multiple administration stage. The indicators of effectiveness include objective response rate (ORR), progression-free survival (PFS), overall survival (OS), disease control rate (DCR), and duration of response (DOR). (2) Safety evaluation: including evaluation of vital signs and physical examinations, electrocardiograms, ECOG PS scores, laboratory tests, adverse events, and the like.

### 2. Clinical trial results:

### 2.1 Results of effectiveness evaluation

Based on the efficacy data obtained for different solid tumors, compound B exhibited good efficacy on patients with solid tumors. Particularly, compound B exhibited clinical benefits for esophageal cancer and squamous cell carcinoma (e.g., esophageal squamous cell carcinoma, pulmonary squamous cell carcinoma, nasopharyngeal squamous cell carcinoma, etc.), especially esophageal squamous cell carcinoma.

### 2.1.1 Efficacy data:

A total of 83 patients with solid tumors were subjected to drug administration, including 48 patients with squamous cell carcinoma, specifically, 41 patients with esophageal squamous cell carcinoma, 5 patients with pulmonary squamous cell carcinoma, and 2 patients with nasopharyngeal squamous cell carcinoma. Upon evaluation, of the 41 patients with esophageal squamous cell carcinoma, 6 cases achieved PR, and 21 cases achieved SD (where 11 cases of SD showed target lesion reduction); of the 5 patients with pulmonary squamous cell carcinoma, 4 cases achieved SD, all of which showed target lesion reduction; of the 2 patients with nasopharyngeal squamous cell carcinoma, 1 case achieved SD, which also showed target lesion reduction.

### 2.2 Typical cases

### (1) Typical case 1

Subject S03002, male, 57 years old, was diagnosed with esophageal squamous cell carcinoma on August 23, 2020. The subject participated in a clinical trial of "toripalimab/placebo + paclitaxel injection + cisplatin injection" in September, 2020. The subject was subjected to a treatment using "toripalimab/placebo + paclitaxel injection + cisplatin injection" from September 24, 2020 to January, 2021, and then subjected to a treatment using "toripalimab/placebo" from February, 2021 to April, 2021, achieving a best response of SD. The subject was withdrawn from the trial due to the efficacy evaluation of PD on April 20, 2021.

The subject signed ICF on April 21, 2021, was staged as IV, and initiated the study drug for the first time on April 30, 2021; the study drug was administered continuously at 6 mg, QD.

Throughout treatment, all of post-cycle efficacy assessments at the end of the first, second, and third cycles indicated PR, with the best overall response recorded as PR.

### (2) Typical case 2

Subject S03008, male, 68 years old, was diagnosed with esophageal squamous cell carcinoma on August 30, 2019. The subject participated in a clinical trial of "sintilimab/placebo + paclitaxel + cisplatin" from October 17, 2019 to December, 2021, achieving a best response of PR. In December, 2021, the efficacy was assessed as PD.

The subject signed ICF on January 13, 2022, was staged as IV, and initiated the study drug for the first time on February 9, 2022; the study drug was administered continuously at 6 mg, QD.

Throughout treatment, the subject achieved SD at the end of the first cycle and PR at the end of the second, third, and fifth cycles, with a confirmed best overall response of PR. The study drug treatment is ongoing.

As of the latest assessment, the subject remains in PR and is currently in the 18th treatment cycle.

### (3) Typical case 3

Subject S03009, male, 49 years old, was diagnosed with stage III esophageal squamous cell carcinoma in January, 2021. From February, 2021, the subject was subjected to a treatment using "albumin-bound paclitaxel in combination with nedaplatin" for 3 cycles, with the best efficacy unknown. From April, 2021, the subject participated in a clinical trial of "tislelizumab/placebo + concurrent chemoradiotherapy (chemotherapeutic drugs: paclitaxel + cisplatin for injection)". In December, 2021, the efficacy was assessed as PD.

The subject signed ICF on February 9, 2022, was staged as IV, and initiated the study drug for the first time on February 21, 2022; the study drug was administered continuously at 9 mg, QD.

Throughout treatment, all of post-cycle efficacy assessments at the end of the first, second, and third cycles indicated PR, with the best overall response recorded as PR.

### (4) Typical case 4

Subject S03018, male, 64 years old, was diagnosed with esophageal squamous cell carcinoma on February 10, 2022. From February 19, 2022 to April 26, 2022, the subject was subjected to a treatment using "albumin-bound paclitaxel + lobaplatin + camrelizumab" for 2 cycles, achieving a best response of SD. Then, the treatment was discontinued due to intolerance. On July 5, 2022, the efficacy was assessed as PD.

The subject signed ICF on July 19, 2022, was staged as IV, and initiated the study drug for the first time on August 1, 2022; the study drug was administered continuously at 6 mg, QD. Throughout treatment, the subject achieved SD at the end of the first cycle and PR at the end of the second and third cycles, with a confirmed best overall response of PR.

### (5) Typical case 5

Subject S03025, male, 66 years old, was diagnosed with esophageal squamous cell carcinoma on August 1, 2017. From August 8, 2019 to November 20, 2019, the subject was subjected to a treatment using "toripalimab injection/placebo + paclitaxel + cisplatin" for 6 cycles, achieving a best response of SD. From December 11, 2019 to August 10, 2021, the subject continued to be subjected to a maintenance therapy using "toripalimab injection/placebo" for 25 cycles, achieving a best response of SD. From December 8, 2021 to March 22, 2022, the subject was further subjected to a treatment using "paclitaxel polymer micelles" for 6 cycles, achieving a best response of SD. On January 11, 2023, the efficacy was assessed as PD.

The subject signed ICF on January 13, 2023, was staged as IV, and initiated the study drug for the first time on February 7, 2023; the study drug was administered continuously at 6 mg, QD. Throughout treatment, the subject achieved SD at the end of the first cycle and PR at the end of the second, third, and fifth cycles, with a confirmed best overall response of PR. The study drug treatment is ongoing.

### (6) Typical case 6

Subject S03033, male, 50 years old, was diagnosed with esophageal squamous cell carcinoma on August 5, 2020. From October 20, 2022 to March 23, 2023, the subject was subjected to a treatment using "paclitaxel + nedaplatin" for 5 cycles, achieving a best response of PR. From April 12, 2023 to May 10, 2023, the subject was subjected to a treatment using "sintilimab" for 2 cycles, achieving a best response of SD. On May 20, 2023, the efficacy was assessed as PD.

The subject signed ICF on May 25, 2023, was staged as IV, and initiated the study drug for the first time on June 5, 2023; the study drug was administered at 6 mg, QD, following the regimen of 3-week administration and then 1-week interruption. Throughout treatment, the subject achieved PR at the end of the first cycle. The study drug treatment is ongoing.

### (7) Typical case 7

Subject S01001, male, 40 years old, was diagnosed with nasopharyngeal squamous cell carcinoma on June 1, 2017. From June 23, 2017 to July 17, 2017, the subject was subjected to a treatment using "paclitaxel + cisplatin" for 2 cycles, achieving a best response of SD. From August 4, 2017 to August 9, 2017, the subject was subjected to a treatment using "gemcitabine + ifosfamide" for 1 cycle, achieving a best response of SD. From September 18, 2017 to November 18, 2017, radiotherapy was given to the "neck and cervical lymph nodes", achieving a best response of PR; from September 18, 2017 to October 30, 2017, the subject was subjected to a concurrent treatment using "cisplatin" for 2 cycles, achieving a best response of SD. From April 27, 2018 to May 4, 2018, radiotherapy was given to the "thoracic spine"; from November 7, 2019 to January 16, 2020, the subject was subjected to a treatment using "geptanolimab injectable liquid (PD-1 monoclonal antibody)" for 6 cycles, achieving a best response of SD. In June, 2020, the efficacy was assessed as PD.

The subject signed ICF on August 19, 2020, was staged as IV, and initiated the study drug for the first time on September 11, 2020; the study drug was administered at 2 mg (dose escalation), QD. Throughout treatment, a best response of SD was achieved, with a maximum reduction of 7.9% in the target lesion. The SD duration was 24 months.

### (8) Typical case 8

Subject S09009, male, 57 years old, was diagnosed with pulmonary squamous cell carcinoma on February 7, 2020. From May 11, 2020 to June 25, 2020, radiotherapy was given to the "right hilum pulmonis + hilar lymph node metastasis". From May 11, 2020, the subject was subjected to a concurrent treatment using "cisplatin + etoposide", achieving a best response of SD; from July 23, 2020 to November 25, 2022, the subject was subjected to a treatment using "sintilimab" for 32 cycles, achieving a best response of SD. In February, 2023, the efficacy was assessed as PD.

The subject signed ICF on February 3, 2023, was staged as IV, and initiated the study drug for the first time on February 10, 2023; the study drug was administered continuously at 4 mg, QD. Throughout treatment, a best response of SD was achieved, with a maximum reduction of 10.7% in the target lesion. The SD duration has lasted for 4 months and is ongoing.

### 2.3 Results of safety evaluation

The monotherapy using compound B was overall safe and controllable. Common (≥ 10%) adverse effects included proteinuria, hypertension, hypoalbuminemia, decreased platelet count, decreased leukocyte count, increased alanine aminotransferase, increased aspartate aminotransferase, hyperuricemia, rash, anemia and fatigue. Most adverse effects were grade 2 or below in severity, required no treatment, and resulted in improvement or recovery, indicating that the adverse effects of compound B were relatively mild. The types of adverse effects of compound B were comparable to those of other protein kinase inhibitors in the same category, but the severity of the adverse effects was relatively mild, with the incidence rate of adverse effects of grade 3 or above being relatively low. Compared with other products in the same category: based on clinical study results of several published related protein kinase inhibitors (see the table below).

**Table 8 Safety evaluation of related protein kinase inhibitors**

| Generic name of drug | Dose of administration (/day) | Common adverse effects (incidence rate ≥10%) | Other risks | Status of approval |
|---|---|---|---|---|
| Surufatinib | 300mg | Proteinuria, hypertension, blood bilirubin increased, diarrhea, hypoalbuminemia, hypertriglyceridemia, thyroid stimulating hormone increased, fatigue/ asthenia, abdominal pain, peripheral edema, hyperuricemia, hemorrhage, etc. | Renal function impairment, arterial/venous thrombosis, gastrointestinal perforation, delayed wound healing, etc. | Approved in China in December, 2020; indication: extrapancreatic neuroendocrine tumors |
| Anlotinib | 12mg | Hypertension*, fatigue*, palmar-plantar erythrodysesthesia syndrome*, hypertriglyceridemia*, proteinuria*, diarrhea, anorexia *, thyroid stimulating hormone increased, hypercholesterolemia, hypothyroidism, etc. | Hemorrhage, pneumothorax, gingival and oral cavity swelling and pain, QT interval prolonged, wound healing delayed, etc. | Approved in China in May, 2018; indications: |
| | | | | lung cancer and soft tissue sarcoma |

| | | | | |
|---|---|---|---|---|
| Note: * indicates that the severity of this adverse effect can reach grade 3 to grade 4 in some cases. | | | | |

The English full names of the abbreviations used in the specification of the present application are listed in Table 9 below:

**Table 9**

| Abbreviation and term | English full name |
|---|---|
| ALT | Alanine aminotransferase |
| AST | Aspartate aminotransferase |
| AE | Adverse event |
| HB | Hemoglobin |
| ULN | Upper limit of normal |
| OS | Overall survival |
| ORR | Objective response rate |
| PK | Pharmacokinetic |
| DLT | Dose-limiting toxicity |
| MTD | Maximum tolerated dose |
| CR | Complete remission |
| PR | Partial remission |
| PD | Progressive disease |
| QD | Quaque die |
| SD | Stable disease |
| DCR | Disease control rate |
| DOR | Duration of response |
| CT | Computer tomography |
| TEAE | Treatment emergent adverse event |
| ICF | Informed consent form |

The embodiments of the present disclosure have been described above. However, the present disclosure is not limited to the embodiments described above. Any modification, equivalent replacement, improvement, and the like made without departing from the spirit and principle of the present disclosure shall fall within the protection scope of the present disclosure.

## Claims

1. Use of compound A or a pharmaceutically acceptable salt thereof in the manufacture of a medicament for treating a tumor, wherein the tumor is esophageal cancer or carcinoma at the esophagogastric junction,

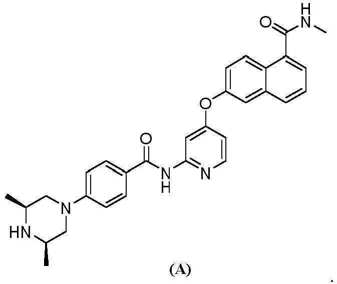

2. The use as claimed in claim 1, wherein the tumor is esophageal squamous cell carcinoma, esophageal adenocarcinoma, squamous cell carcinoma at the esophagogastric junction, or adenocarcinoma at the esophagogastric junction; preferably, the tumor is esophageal squamous cell carcinoma or squamous cell carcinoma at the esophagogastric junction.

3. The use as claimed in claim 1 or 2, wherein the tumor is a locally advanced tumor or a metastatic tumor, preferably an unresectable locally advanced tumor or metastatic tumor.

4. The use as claimed in any one of claims 1-3, wherein the tumor is a tumor that has failed a prior treatment.

5. The use as claimed in any one of claims 1-4, wherein the tumor is a tumor that has failed a systemic drug treatment.

6. The use as claimed in any one of claims 1-5, wherein the tumor is a tumor that has failed a first-line treatment, a second-line treatment, or a later-line treatment.

7. The use as claimed in any one of claims 1-6, wherein the tumor is a tumor that has failed treatment with one, two, three, or more of an immunotherapeutic drug, a chemotherapeutic drug, radiotherapy, biological therapy, traditional Chinese medicine therapy, and a small molecule targeted drug, preferably a tumor that has failed treatment with radiotherapy, an immunotherapeutic drug, or a chemotherapeutic drug, further preferably a tumor that has failed treatment with radiotherapy and an immunotherapeutic drug, further preferably a tumor that has failed treatment with radiotherapy and a chemotherapeutic drug, further preferably a tumor that has failed treatment with a chemotherapeutic drug and an immunotherapeutic drug, and further preferably a tumor that has failed treatment with a chemotherapeutic drug, immunotherapy, and radiotherapy.

8. Use of compound A or a pharmaceutically acceptable salt thereof in the manufacture of a medicament for treating a tumor, 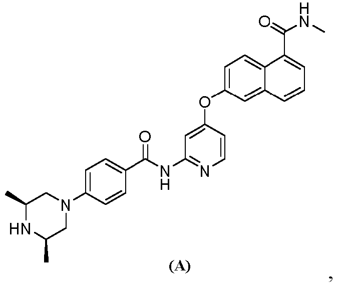 wherein the tumor is squamous cell carcinoma.

9. The use as claimed in claim 8, wherein the tumor is selected from head and neck squamous cell carcinoma, digestive tract squamous cell carcinoma, and respiratory tract squamous cell carcinoma; preferably, the tumor is selected from head and neck squamous cell carcinoma, upper digestive tract squamous cell carcinoma, and respiratory squamous cell carcinoma; further preferably, the tumor is selected from esophageal squamous cell carcinoma, squamous cell carcinoma at the esophagogastric junction, head and neck squamous cell carcinoma, and pulmonary squamous cell carcinoma; further preferably, the tumor is selected from esophageal squamous cell carcinoma, squamous cell carcinoma at the esophagogastric junction, nasopharyngeal squamous cell carcinoma, and pulmonary squamous cell carcinoma; further preferably, the tumor is selected from nasopharyngeal squamous cell carcinoma, esophageal squamous cell carcinoma, and pulmonary squamous cell carcinoma; further preferably, the tumor is esophageal squamous cell carcinoma.

10. The use as claimed in claim 8 or 9, wherein the tumor is a locally advanced tumor or a metastatic tumor, preferably an unresectable locally advanced tumor or metastatic tumor.

11. The use as claimed in any one of claims 8-10, wherein the tumor is a tumor that has failed a prior treatment.

12. The use as claimed in any one of claims 8-11, wherein the tumor is a tumor that has failed a systemic drug treatment.

13. The use as claimed in any one of claims 8-12, wherein the tumor is a tumor that has failed a first-line treatment, a second-line treatment, or a later-line treatment.

14. The use as claimed in any one of claims 8-13, wherein the tumor is a tumor that has failed treatment with one, two, three, or more of an immunotherapeutic drug, a chemotherapeutic drug, radiotherapy, biological therapy, traditional Chinese medicine therapy, and a small molecule targeted drug.

15. The use as claimed in claim 7 or 14, wherein the chemotherapeutic drug is selected from a cytotoxic drug, an antimetabolite drug, an antibiotic drug, an alkaloid drug, a DNA topoisomerase inhibitor chemotherapeutic drug, a microtubule-targeting chemotherapeutic drug, a platinum-based chemotherapeutic drug, and a hormone drug; preferably, the chemotherapeutic drug is selected from an antimetabolite drug, an alkaloid drug, a DNA topoisomerase inhibitor chemotherapeutic drug, a microtubule-targeting chemotherapeutic drug, and a platinum-based chemotherapeutic drug.

16. The use as claimed in claim 7, 14, or 15, wherein the chemotherapeutic drug is selected from a taxane-based drug, a platinum-based drug, a fluorouracil-based drug, and a DNA topoisomerase inhibitor chemotherapeutic drug; preferably, the taxane-based drug is selected from paclitaxel, docetaxel, and cabazitaxel; preferably, the DNA topoisomerase inhibitor chemotherapeutic drug is selected from hydroxycamptothecin, irinotecan, topotecan, etoposide, and teniposide; preferably, the platinum-based drug is selected from cisplatin, carboplatin, sulfatodiamino cyclohexane platinum, lobaplatin, oxaliplatin, and nedaplatin, preferably cisplatin, oxaliplatin, or nedaplatin; further preferably, the chemotherapeutic drug is selected from paclitaxel, cisplatin, and nedaplatin.

17. The use as claimed in claim 7 or 14, wherein the immunotherapeutic drug is selected from an anti-PD-1 antibody, an anti-PD-L1 antibody, and an antibody-drug conjugate; preferably, the antibody-drug conjugate is an anti-HER2 antibody-drug conjugate; preferably, the anti-PD-1 antibody is selected from pembrolizumab, camrelizumab, tislelizumab, nivolumab, cemiplimab, sintilimab, and toripalimab; preferably, the anti-PD-1 antibody is selected from toripalimab, sintilimab, and tislelizumab; preferably, the anti-PD-L1 antibody is selected from atezolizumab, avelumab, and durvalumab; preferably, the anti-HER2 antibody-drug conjugate is selected from trastuzumab and disitamab.

18. The use as claimed in any one of claims 1-17, wherein the tumor is a tumor that has failed treatment with a chemotherapeutic drug and an immunotherapeutic drug, preferably a tumor that has failed treatment with a taxane-based chemotherapeutic drug + a platinum-based chemotherapeutic drug + an anti-PD-1 antibody, and further preferably a tumor that has failed treatment with one, two, three, or more of toripalimab + paclitaxel + cisplatin, sintilimab + paclitaxel + cisplatin, paclitaxel + lobaplatin + camrelizumab, paclitaxel + nedaplatin, and sintilimab.

19. The use as claimed in any one of claims 1-16, wherein the tumor is a tumor that has failed treatment with a chemotherapeutic drug, preferably a tumor that has failed treatment with a taxane-based chemotherapeutic drug + a platinum-based chemotherapeutic drug, and further preferably a tumor that has failed treatment with paclitaxel + cisplatin or paclitaxel + nedaplatin.

20. The use as claimed in any one of claims 1-17, wherein the tumor is a tumor that has failed treatment with a chemotherapeutic drug, immunotherapy, and radiotherapy, preferably a tumor that has failed treatment with a taxane-based chemotherapeutic drug + a platinum-based chemotherapeutic drug + radiotherapy + an anti-PD-1 antibody, and further preferably a tumor that has failed treatment with tislelizumab + radiotherapy + paclitaxel + cisplatin.

21. The use as claimed in any one of claims 1-20, wherein the compound A or the pharmaceutically acceptable salt thereof is administered at a daily dose of 1-50 mg, preferably 1-30 mg, further preferably 1-25 mg, further preferably 1-20 mg, further preferably 2-18 mg, further preferably 4-16 mg, further preferably 5-15 mg, further preferably 4-10 mg, further preferably 4-9 mg, further preferably 2-9 mg, further preferably 2-6 mg, further preferably 5-9 mg, further preferably 6-9 mg, further preferably 6-8 mg, further preferably 6-12 mg, further preferably 6-10 mg, and further preferably 2 mg, 3 mg, 4 mg, 5 mg, 6 mg, 7 mg, 8 mg, 9 mg, or 10 mg.

22. The use as claimed in any one of claims 1-21, wherein the compound A or the pharmaceutically acceptable salt thereof is administered at a frequency of once, twice, three times, or more times daily.

23. The use as claimed in any one of claims 1-22, wherein the compound A or the pharmaceutically acceptable salt thereof is given according to a continuous administration regimen or an intermittent administration regimen; preferably, the intermittent administration regimen comprises an administration period and an interruption period.

24. The use as claimed in claim 23, wherein the compound A or the pharmaceutically acceptable salt thereof is administered according to a method as follows: 3-week continuous administration, followed by 1-week interruption.

25. The use as claimed in any one of claims 1-24, wherein the medicament is further used in combination with one, two, three, or more of drugs commonly used clinically for treating esophageal cancer, carcinoma at the esophagogastric junction, or squamous cell carcinoma.

26. The use as claimed in any one of claims 1-25, wherein the pharmaceutically acceptable salt is selected from hydrochloride, hydrobromide, nitrate, sulfate, phosphate, formate, acetate, propionate, benzoate, maleate, fumarate, succinate, tartrate, citrate, methanesulfonate, ethanesulfonate, benzenesulfonate, and *p*-toluenesulfonate, preferably hydrochloride, hydrobromide, maleate, phosphate, tartrate, fumarate, and succinate, further preferably hydrochloride, maleate, phosphate, tartrate, and fumarate, and further preferably hydrochloride.

27. The use as claimed in any one of claims 1-26, wherein the pharmaceutically acceptable salt of compound A is compound B:

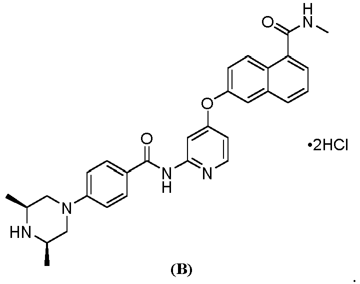

28. The use as claimed in claim 27, wherein the compound B is in a solid form, preferably in a crystalline form.

29. The use as claimed in claim 27 or 28, wherein the crystalline form of compound B is a crystalline form I having characteristic peaks in an X-ray powder diffraction pattern using Cu-Kα radiation at the following 2θ angles: 5.0±0.2°, 13.5±0.2°, 19.3±0.2°, 20.5±0.2°, 21.4±0.2°, and 25.2±0.2°.

30. A pharmaceutical composition for treating a tumor, comprising the compound A or the pharmaceutically acceptable salt thereof as claimed in any one of claims 1-26 or the compound B as claimed in any one of claims 27-29, and optionally a pharmaceutically acceptable carrier, wherein the tumor is esophageal cancer, carcinoma at the esophagogastric junction, or squamous cell carcinoma.

31. The pharmaceutical composition as claimed in claim 30, comprising the compound A or the pharmaceutically acceptable salt thereof and the pharmaceutically acceptable carrier, and further comprising a therapeutically effective amount of an additional drug, wherein the additional drug is selected from one, two, three, or more of drugs commonly used clinically for treating esophageal cancer, carcinoma at the esophagogastric junction, and squamous cell carcinoma.

32. The pharmaceutical composition as claimed in claim 30 or 31, comprising the compound B and the pharmaceutically acceptable carrier, and further comprising a therapeutically effective amount of an additional drug, wherein the additional drug is selected from one, two, three, or more of drugs commonly used clinically for treating esophageal cancer, carcinoma at the esophagogastric junction, and squamous cell carcinoma.

33. A pharmaceutical kit, comprising (a) at least one unit dose of the pharmaceutical composition as claimed in any one of claims 30-32 and (b) instructions for use in treating a tumor, wherein the tumor is esophageal cancer, carcinoma at the esophagogastric junction, or squamous cell carcinoma.
